# FASCICULE DE BREVET EUROPEEN

(11) **EP 1 874 777 B1**
(45) Date de publication et mention de la délivrance du brevet: **08.06.2011**
(21) Numéro de dépôt: 06743712.9
(22) Date de dépôt: 18.04.2006
(51) Int. Cl.: C07D 487/04, C07D 209/12, A61K 31/519

(54) **DÉRIVÉS DE 1H-PYRIMIDO[4,5-B]INDOLE, LEUR PRÉPARATION ET LEUR APPLICATION EN THÉRAPEUTIQUE**
1H-PYRIMIDO[4,5-B]INDOL-DERIVATE, DEREN HERSTELLUNG UND THERAPEUTISCHE ANWENDUNG
1H-PYRIMIDO[4,5-B]INDOLE DERIVATIVES, THEIR PREPARATION AND THERAPEUTIC USE

(30) Priorité: 20.04.2005 FR 0503934
(43) Date de publication de la demande: 09.01.2008
(73) Titulaire: Sanofi-Aventis, 75013 Paris (FR)
(72) Inventeur: OLIVIER-BANDINI, Anne, 75012 Paris (FR); MARGUET, Frank, F-91370 Verrières le Buisson (FR); FROISSANT, Jacques, 41160 Brevainville (FR); PUECH, Frédéric c/o sanofi-aventis Département Brevets, F-75013 PARIS (FR)
(74) Mandataire: Quellari, Mylène Audrey Séverine
(86) Numéro de dépôt international: PCT/FR2006/000843
(87) Numéro de publication internationale: WO 2006/111648

(56) Documents cités:
- WO-A-93/20078
- WO-A-96/26941
- DOTZAUER, BERND ET AL: "Synthesis of medicinally interesting 2,4-diamino-9H-pyrimido[4,5-b]indol-6- ols via extension of the Nenitzescu reaction" SYNLETT , (6), 1039-1043 CODEN: SYNLES; ISSN: 0936-5214, 2004, XP009058869
- BUNDY, GORDON L. ET AL: "Synthesis of Novel 2,4-Diaminopyrrolo[2,3-d]pyrimidines with Antioxidant, Neuroprotective, and Antiasthma Activity" JOURNAL OF MEDICINAL CHEMISTRY , 38(21), 4161-3 CODEN: JMCMAR; ISSN: 0022-2623, 1995, XP001106595
- BUNDY, GORDON L. ET AL: "Synthesis of 2,4-Di-1-pyrrolidinyl-9H-pyrimido[4,5-b]in doles, Including Antiasthma Clinical Candidate PNU-142731A" ORGANIC PROCESS RESEARCH & DEVELOPMENT , 5(2), 144-151 CODEN: OPRDFK; ISSN: 1083-6160, 2001, XP009058870
- DATABASE CA [Online] CHEMICAL ABSTRACTS SERVICE, COLUMBUS, OHIO, US; KONDO, YOSHINORI ET AL: "Condensed heteroaromatic ring systems. XVI. Synthesis of pyrrolo[2,3-d]pyrimidine derivatives" XP002392425 extrait de STN Database accession no. 1990:459080 & CHEMICAL & PHARMACEUTICAL BULLETIN , 37(11), 2933-6 CODEN: CPBTAL; ISSN: 0009-2363, 1989,

## Description

L'invention a pour objet des composés dérivés de 1*H*-pyrimido[4,5-*b*]indole.

La demande internationale de brevet WO96/26941 décrit des dérivés de pyrimido|4,5-*b*]indoles ; la demande internationale de brevet WO93/20078 décrit des amines bicycliques et hétérocycliques. Dotzauer et al. (2004) Synlett 6: 1039-1043 décrit la synthèse de dérivés de 2,4-diamino-9H-pyrimido[4,5-*b*]indoles. Dans Bundy et al. (1995) J. Med Chem. 38(21): 4161-4163, est rapportée la synthèse de 2,4-diaminopyrrolo[2,3-*d*]pyrimidines. Bundy et al. (2001) Organic Process Research and Development 5(2) : 144-151 décrit la synthèse de 2,4-di-1-pyrrolidinyl-9H-pyrimido[4,5-*b*]indoles. Sont également connus des dérivés de pyrrolo[2,3-*d*]pyrimidine (Kondo et al. Chemical & Pharmaceutical Bulletin (1989) 37(11) : 2933-2936).

Un premier objet de l'invention concerne les composés répondant à la formule générale (I) ci-après.

Un autre objet de l'invention concerne des procédés de préparation des composés de formule générale (I).

Un autre objet de l'invention concerne des composés utilisables notamment en tant qu'intermédiaires de synthèse des composés de formule générale (I).

Un autre objet de l'invention concerne les utilisations des composés de formule générale (I) notamment dans des médicaments ou dans des compositions pharmaceutiques.

Les composés de l'invention répondent à la formule générale (I) : 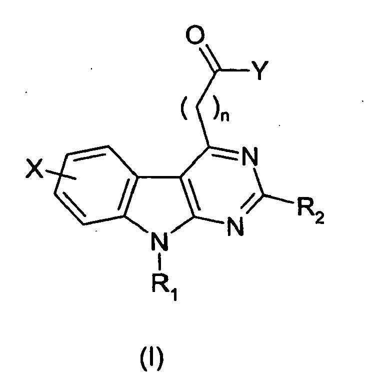 dans laquelle
n représente le chiffre 0 ou 1 ;
X représente un atome d'hydrogène ou d'halogène ;
Y représente un groupe OR₃ ou un groupe NR₄R₅ ;
R₁ représente un atome d'hydrogène ou un groupe (C₁-C₆)alkyle;
R₂ représente un groupe (C₁-C₆)alkyle, un phényle ou un hétérocycle monocyclique ou bicyclique, lesdits groupes phényle ou hétérocycle pouvant porter un ou plusieurs atomes d'halogène et/ou un ou plusieurs groupes (C₁-C₆)alkyle, (C₁-C₆)alcoxyle, (C₁-C₆)alcoxy-(C₁-C₆)alkyle, (C₁-C₆)alkylamino-(C₁-C₆)alkyle ou (C₁-C₆)dialkylamino-(C₁-C₆)alkyle ;
R₃ représente un atome d'hydrogène, un groupe (C₁-C₆)alkyle ou un benzyle ;
R₄ et R₅ représentent chacun, indépendamment l'un de l'autre, un atome d'hydrogène ou un groupe (C₁-C₆)alkyle, ou bien R₄ et R₅ forment, avec l'atome d'azote qui les porte, un groupe aziridinyle, azétidinyle, pyrrolidinyle, pipéridinyle, morpholinyle, thiomorpholinyle, ou pipérazinyle, éventuellement substitué par un (C₁-C₆)alkyle, phényle ou hétérocycle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention :
- un atome d'halogène représente un fluor, un chlore, un brome ou un iode ;
- (Cₜ-C_{z}) où t et z peuvent prendre les valeurs de 1 à 6, représente une chaîne carbonée pouvant avoir de 1 à 6 atomes de carbone ;
- un groupe alkyle représente un groupe aliphatique saturé linéaire, ramifié ou cyclique ou leur combinaison, éventuellement substitué par un ou plusieurs atomes d'halogène. A titre d'exemples, on peut citer les groupes méthyle, éthyle, propyle, isopropyle, butyle, isobutyle, terbutyle, pentyle, cyclopropyle, méthylcyclopropyle, cyclopropylméthyle, cyclobutyle, cyclopentyle, cyclohexyle, fluorométhyle, trifluorométhyle;
- un groupe alcoxyle représente un atome d'oxygène substitué par un groupe alkyle où le groupe alkyle est tel que défini précédemment. A titre d'exemple, on peut citer le groupe méthoxyle.
- un groupe alkylamino représente une amine substituée par un groupe alkyle où le groupe alkyle est tel que défini précédemment. A titre d'exemple, on peut citer le groupe méthylamino.
- un groupe alkylamino-alkyle représente un groupe alkyle substitué par un groupe alkylamino. A titre d'exemple, on peut citer le groupe (méthylamino)-méthyle.
- un groupe dialkylamino représente une amine substituée par deux groupes alkyles, où chaque groupe alkyle est tel que défini précédemment. A titre d'exemple, on peut citer le groupe diméthylamino.
- un groupe dialkylamino-alkyle représente un groupe alkyle substitué par un groupe dialkylamino. A titre d'exemple, on peut citer le groupe (diméthylamino)-méthyle.
- un groupe alcoxy-alkyle représente un groupe alkyle substitué par un groupe alcoxyle. A titre d'exemples, on peut citer les groupes méthoxyméthyle, éthoxyméthyle, méthoxyéthyle.
- un hétérocycle représente un cycle saturé, partiellement saturé ou aromatique de 4 à 12 chaînons possédant au moins un atome choisi parmi O, S ou N. A titre d'exemples d'hétérocycles monocycliques, on peut citer les groupes pyridinyle, pyrimidinyle, pyrazinyle, pyridazinyle, thiazolyle, furanyle, thiophènyle, tétrahydropyranyle. A titre d'exemples d'hétérocycliques bicycliques, on peut citer les groupes quinolinyle, dihydroquinolinyle, tétrahydroquinolinyle, isoquinolinyle, quinoxalinyle, quinazolinyle, phtalazinyle, cinnolinyle, benzothiazolyle, benzofuranyle, benzothiophènyle.

Un premier sous-groupe de composés de formule générale (I) est constitué par les composés pour lesquels :
n représente le chiffre 0 ou 1 ;
X représente un atome d'hydrogène ou d'halogène ;
Y représente un groupe OR₃ ou un groupe NR₄R₅ ;
R₁ représente un atome d'hydrogène ou un groupe (C₁-C₆)alkyle ;
R₂ représente un phényle ou un hétérocycle de type pyridinyle, pyrimidinyle, pyrazinyle, pyridazinyle, le phényle ou l'hétérocycle pouvant porter un ou plusieurs atomes d'halogène et/ou un ou plusieurs groupes (C₁-C₆)alkyle, (C₁-C₆)alcoxyle ;
R₃ représente un hydrogène, un (C₁-C₆)alkyle ou un benzyle ; et
R₄ et R₅ représentent chacun, indépendamment l'un de l'autre, un atome d'hydrogène ou un groupe (C₁-C₆)alkyle, ou bien R₄ et R₅ forment, avec l'atome d'azote qui les porte, un groupe aziridinyle, azétidinyle, pyrrolidinyle, pipéridinyle, morpholinyle, thiomorpholinyle, ou pipérazinyle, éventuellement substitué par un (C₁-C₆)alkyle, phényle ou azétidinyle.

Un deuxième sous-groupe de composés de formule générale (I) est constitué par les composés pour lesquels :
n représente le chiffre 0 ou 1 ; et/ou
X représente un atome d'hydrogène ou d'halogène, plus particulièrement un chlore ou un fluor ; et/ou
Y représente un groupe OR₃ ou un groupe NR₄R₅ ; et/ou
R₁ représente un atome d'hydrogène ou un groupe (C₁-C₆)alkyle, plus particulièrement un méthyle ; et/ou
R₂ représente un phényle ou un hétérocycle de type pyridinyle ou pyrazinyle, le phényle ou l'hétérocyclique pouvant porter un ou plusieurs groupes (C₁-C₆)alkyle, plus particulièrement méthyle, ou (C₁-C₆)alcoxyle, plus particulièrement méthoxyle ; et/ou
R₃ représente un hydrogène ou un (C₁-C₆)alkyle, plus particulièrement un méthyle ou un éthyle ; et/ou
R₄ et R₅ représentent chacun, indépendamment l'un de l'autre, un groupe (C₁-C₆)alkyle, plus particulièrement un méthyle ou un éthyle, ou bien R₄ et R₅ forment, avec l'atome d'azote qui les porte, un groupe pyrrolidinyle, pipéridinyle, morpholinyle ou pipérazinyle, éventuellement substitué par un (C₁-C₆)alkyle, plus particulièrement méthyle, ou un azétidinyle.

Un troisième sous-groupe de composés de formule générale (I) est constitué par les composés pour lesquels :
n représente le chiffre 0 ou 1;
X représente un atome d'hydrogène ou de fluor ou de chlore ;
Y représente un groupe hydroxy, OCH₃, O(CH₂CH₃), N(CH₃)₂, N(CH₂CH₃)₂, pyrrolidinyle, pipéridinyle, morpholinyle, (N-azétidinyl)-pipéridinyle ou N-méthylpipérazinyle;
R₁ représente un atome d'hydrogène, un groupe méthyle ou isobutyle ;
R₂ représente un phényle, un groupe méthyle, isopropyle, cyclopropyle ou un hétérocycle de type pyridinyle, pyrazinyle, pyrimidinyle, thiazolyle, tétrahydroquinolinyle, tétrahydropyranyle, les groupes phényle et hétérocycle pouvant éventuellement être substitués par un ou plusieurs groupes halogène et/ou un ou plusieurs groupes (C₁-C₆)alkyle, (C₁-C₆)alcoxyle, (C₁-C₆)alcoxy-(C₁-C₆)alkyle, (C₁-C₆)dialkylamino, (C₁-C₆)dialkylamino-(C₁-C₆)alkyle.

Parmi les composés de formule (I) objets de l'invention, on peut notamment citer les composés suivants :
1. *N*,*N*,9-triméthyl-2-phényl-9*H*-pyrimido[4,5-*b*]indole-4-carboxamide
2. 7-chloro-*N*,*N*,9-triméthyl-2-phényl-9*H*-pyrimido[4,5-*b*]indole-4-carboxamide
3. 7-fluoro-*N*,*N*,9-triméthyl-2-phényl-9*H*-pyrimido[4,5-*b*]indole-4-carboxamide
4. 6-chloro-*N*,*N*,9-triméthyl-2-phényl-9*H*-pyrimido[4,5-*b*]indole-4-carboxamide
5. 7-chloro-*N*,*N*-diméthyl-2-phényl-9*H*-pyrimido[4,5-*b*]indole-4-carboxamide
6. 7-chloro-*N*,*N*-diéthyl-9-méthyl-2-phényl-9*H*-pyrimido[4,5-*b*]indole-4-carboxamide
7. 7-chloro-9-méthyl-2-phényl-4-(pyrrolidin-1-ylcarbonyl)-9*H*-pyrimido[4,5-*b*]indole
8. 7-chloro-9-méthyl-2-phényl-4-(pipéridin-1-ylcarbonyl)-9*H*-pyrimido[4,5-*b*]indole
9. 7-chloro-9-méthyl-4-(morpholin-4-ylcarbonyl)-2-phényl-9*H*-pyrimido[4,5-*b*]indole
10. 4-[(4-azétidin-1-yl)pipéridin-1-ylcarbonyl-7-chloro-9-méthyl-2-phényl-9*H-*pyrimido[4,5-*b*]indole
11. 7-chloro-9-méthyl-4-[(4-méthylpipérazin-1-yl)carbonyl]-2-phényl-9*H*-pyrimido-[4,5-*b*]indole
12. 6-chloro-*N*,*N*,9-triméthyl-2-pyridin-4-yl-9*H*-pyrimido[4,5-*b*]indole-4-carboxamide
13. 7-fluoro-*N,N*,9-triméthyl-2-pyridin-4-yl-9*H*-pyrimido[4,5-*b*]indole-4-carboxamide
14. 7-chloro-9-méthyl-2-pyridin-4-yl-4-(pyrrolidin-1-ylcarbonyl)-9*H*-pyrimido-[4,5-*b*]indole
15. 7-chloro-*N*,*N*,9-triméthyl-2-pyridin-4-yl-9*H*-pyrimido[4,5-*b*]indole-4-carboxamide
16. 7-chloro-*N,N*,9-triméthyl-2-pyridin-3-yl-9*H*-pyrimido[4,5-*b*]indole-4-carboxamide
17. 7-chloro-*N,N*,9-triméthyl-2-pyridin-2-yl-9*H*-pyrimido[4,5-*b*]indole-4-carboxamide
18. 7-chloro-*N,N*,9-triméthyl-2-pyrazin-2-yl-9*H*-pyrimido[4,5-*b*]indole-4-carboxamide
19. 2-(7-chloro-9-méthyl-2-phényl-9*H*-pyrimido[4,5-*b*]indol-4-yl)-*N*,*N*-diméthyl-acétamide
20. 2-(6-chloro-9-méthyl-2-phényl-9*H*-pyrimido[4,5-*b*]indol-4-yl)-*N*,*N*-diméthyl-acétamide
21. 2-(7-chloro-9-méthyl-2-pyridin-4-yl-9*H*-pyrimido[4,5-*b*]indol-4-yl)-*N*,*N*-diméthyl-acétamide
22. 2-(7-chloro-9-méthyl-2-pyridin-3-yl-9*H*-pyrimido[4,5-*b*]indol-4-yl)-*N*,*N*-diméthyl-acétamide
23. *N*,*N*-diméthyl-2-phényl-9*H*-pyrimido[4,5-*b*]indole-4-carboxamide
24. 7-chloro-9-méthyl-2-phényl-9*H*-pyrimido[4,5-*b*]indole-4-carboxylate d'éthyle
25. 7-chloro-9-méthyl-2-phényl-9*H*-pyrimido[4,5-*b*]indole-4-carboxylate de méthyle
26. acide 7-chloro-9-méthyl-2-phényl-9*H*-pyrimido[4,5-*b*]indole-4-carboxylique
27. 7-chloro-*N,N*,9-triméthyl-2-(6-méthylpyridin-3-yl)-9*H*-pyrimido[4,5-*b*]indole-4-carboxamide
28. 7-chloro-2-(6-méthoxypyridin-3-yl)-*N,N*,9-triméthyl-9*H*-pyrimido[4,5-*b*]indole-4-carboxamide
29. 7-chloro-*N,N*,9-triméthyl-2-(2-méthylpyridin-4-yl)-9*H*-pyrimido[4,5-*b*]indole-4-carboxamide
30. 7-chloro-2-(2-méthoxypyridin-4-yl)-*N,N*,9-triméthyl-9*H-*pyrimido[4,5-*b*]indole-4-carboxamide
31. 7-chloro-9-isobutyl-*N*,*N*-diméthyl-2-pyridin-4-yl-9*H*-pyrimido[4,5-*b*]indole-4-carboxamide
32. 7-chloro-2-cyclopropyl-*N*,*N*,9-triméthyl-9*H*-pyrimido[4,5-*b*]indole-4-carboxamide
33. 7-chloro-*N*,*N*,2,9-tétraméthyl-9*H*-pyrimido[4,5-*b*]indole-4-carboxamide
34. 7-chloro-2-isopropyl-*N*,*N*,9-triméthyl-9*H*-pyrimido[4,5-*b*]indole-4-carboxamide
35. 7-chloro-*N*,*N*,9-triméthyl-2-(tétrahydro-2*H*-pyran-4-yl)-9*H*-pyrimido[4,5-*b*]indole-4-carboxamide
36. 7-chloro-2-{4-[(diméthylamino)méthyl]phényl}-*N*,*N*,9-triméthyl-9*H*-pyrimido[4,5-*b*]indole-4-carboxamide
37. 7-chloro-2-(6-chloropyridin-3-yl)-*N*,*N*,9-triméthyl-9*H*-pyrimido[4,5-*b*]indole-4-carboxamide
38. 7-chloro-*N,N*,9-triméthyl-2-[6-(trifluorométhyl)pyridin-3-yl]-9*H*-pyrimido[4,5-*b*]indole-4-carboxamide
39. 7-chloro-2-(6-éthoxypyridin-3-yl)-*N,N*,9-triméthyl-9*H*-pyrimido[4,5-*b*]indole-4-carboxamide
40. 7-chloro-2-(6-éthylpyridin-3-yl)-*N,N*,9-triméthyl-9*H*-pyrimido[4,5-*b*]indole-4-carboxamide
41. 7-chloro-2-(5-éthylpyridin-3-yl)-*N,N*,9-triméthyl-9*H*-pyrimido[4,5-*b*]indole-4-carboxamide
42. 7-chloro-*N,N*,9-triméthyl-2-(5-méthylpyridin-3-yl)-9*H*-pyrimido[4,5-*b*]indole-4-carboxamide
43. 7-chloro-*N,N*,9-triméthyl-2-pyrimidin-5-yl-9*H*-pyrimido[4,5-*b*]indole-4-carboxamide
44. 7-chloro-2-(5-méthoxypyridin-3-yl)-*N,N*,9-triméthyl-9*H*-pyrimido[4,5-*b*]indole-4-carboxamide
45. 7-chloro-2-[6-(méthoxyméthyl)pyridin-3-yl]-*N,N*,9-triméthyl-9*H*-pyrimido[4,5-*b*]indole-4-carboxamide
46. 7-chloro-*N,N*,9-triméthyl-2-(2-méthyl-1,3-thiazol-4-yl)-9*H*-pyrimido[4,5-*b*]indole-4-carboxamide
47. 7-chloro-2-{6-[(diméthylamino)méthyl]pyridin-3-yl}-*N*,*N*,9-triméthyl-9*H-*pyrimido[4,5-*b*]indole-4-carboxamide
48. 7-chloro-2-(5,6-diméthylpyridin-3-yl)-*N*,*N*,9-triméthyl-9*H*-pyrimido[4,5-*b*]indole-4-carboxamide
49. 7-chloro-*N,N*,9-triméthyl-2-(5,6,7,8-tétrahydroquinolin-3-yl)-9*H*-pyrimido[4,5-*b*]indole-4-carboxamide
50. 7-chloro-2-(2,6-diméthylpyridin-4-yl)-*N*,*N*,9-triméthyl-9*H*-pyrimido[4,5-*b*]indole-4-carboxamide

Les composés de formule générale (I) peuvent être préparés par les procédés illustrés par les schémas qui suivent.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

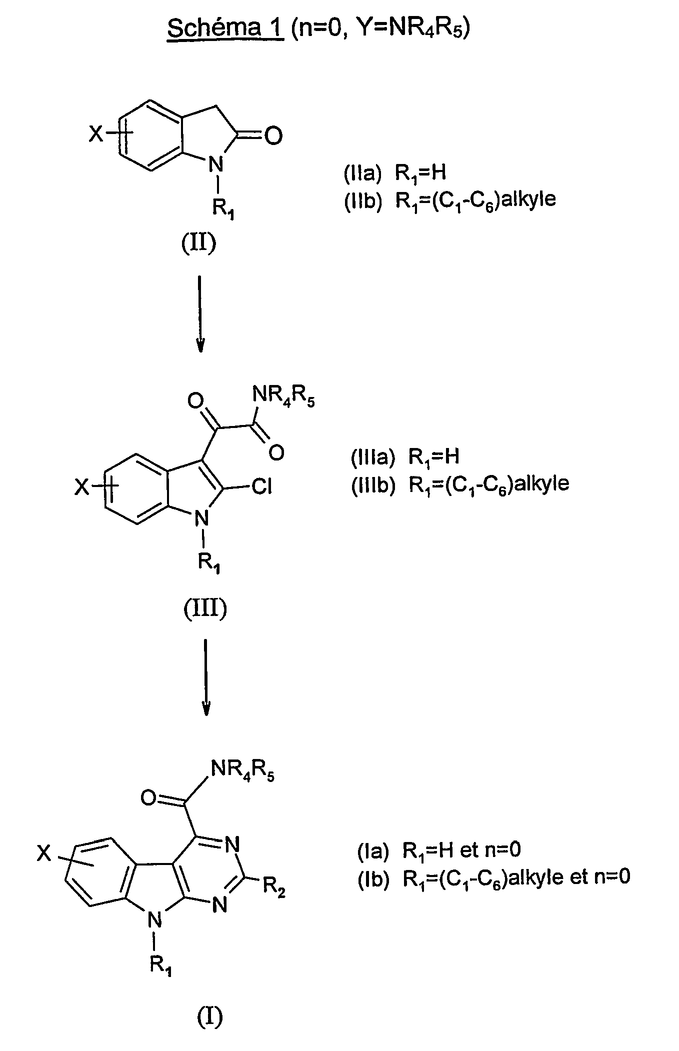

### Voie de synthèse pour les carboxamides (n=0, Y=NR₄R₅).

Comme illustré dans le schéma 1, un 2-oxindole de formule générale (II), dans laquelle X et R₁ sont tels que définis ci-dessus, est transformé en 2-chloroindole de formule générale (III), dans laquelle X, R₁, R₄ et R₅ sont tels que définis ci-dessus, d'abord par action du chlorure d'oxalyle dans un solvant polaire aprotique comme du dichlorométhane par exemple, puis par action d'une amine de formule générale HNR₄R₅, dans laquelle R₄ et R₅ sont tels que définis ci-dessus. Le 2-chloroindole de formule générale (III) est cyclisé en présence d'une amidine d'alkyle, d'hétéroalkyle, d'aryle ou d'hétéroaryle de formule générale R₂C(NH)NH₂ dans laquelle R₂ est tel que défini ci-dessus, en chauffant dans un solvant apolaire ou polaire tel que par exemple du xylène ou de la *N*,*N*-diméthylformamide pour conduire à un composé de formule générale (I) dans laquelle X, R₁, R₂, R₄ et R₅ sont tels que définis ci-dessus.

Les composés (Ib) pour lesquels R₁=(C₁-C₆)alkyle peuvent être obtenus à partir des composés (la) pour lesquels R₁=H par une réaction d'alkylation en utilisant par exemple du 1,1-di(C₁-C₆)alkoxytriméthylamine dans un solvant tel que du toluène à chaud.

Les composés (IIb) pour lesquels R₁=(C₁-C₆)alkyle peuvent être obtenus à partir des composés (IIa) pour lesquels R₁=H par alkylation en utilisant par exemple un dialkylsulfate, par analogie à une procédure décrite dans la littérature (G.W. Rewcastle et al., J. Med. Chem. (1994), 37, 2033).

Les 2-oxindoles de formule générale (IIa) sont connus dans la littérature ou disponibles dans le commerce.

### Voie de synthèse pour les esters carboxyliques (n=0 ; Y=OR₃)

Comme illustré dans le schéma 2, le 2-chloroindole de formule générale (IV), où X et R₁ sont tels que définis ci-dessus, est transformé en pyrimido[4,5-*b*]indol-4-one (V) par traitement avec du chlorure de thionyle, puis par ajout d'une amidine d'alkyle, hétéroalkyle, d'aryle ou d'hétéroaryle de formule générale R₂C(NH)NH₂ dans laquelle R₂ est tel que défini ci-dessus et enfin par chauffage dans un solvant à haut point d'ébullition comme de l'éther de diphényle par exemple. La pyrimido[4,5-*b*]indol-4-one (V) peut également être obtenue à partir du 2-amino-indole de formule générale (VI), où X et R₁ sont tels que définis ci-dessus et R est un (C₁-C₆)alkyle, en le faisant réagir avec un dérivé cyanoaryle ou cyanohétéroaryle de formule générale R₂CN dans laquelle R₂ est tel que défini ci-dessus en présence d'une base telle que de l'hydrure de sodium dans un solvant polaire aprotique comme du tétrahydrofurane, à chaud.

La pyrimido[4,5-*b*]indol-4-one de formule générale (V), dans laquelle X, R₁ et R₂ sont tels que définis ci-dessus, est ensuite transformée en triflate de formule générale (VII), dans laquelle X, R₁ et R₂ sont tels que définis ci-dessus et OTf est un groupement triflate, par exemple en présence d'anhydride trifluorométhanesulfonique dans un solvant tel que le dichlorométhane. L'ester de formule générale (I), dans laquelle X, R₁, R₂ et R₃ sont tels que définis ci-dessus, est finalement obtenu par une réaction de carbonylation du triflate de formule générale (VII), par exemple en présence d'un catalyseur tel que l'acétate de palladium, de monoxyde de carbone, de ligand de type phosphine tel que le 1,3-bis(diphénylphosphinopropane) et d'un alcool de formule générale R₃OH, dans laquelle R₃ est tel que défini ci-dessus.

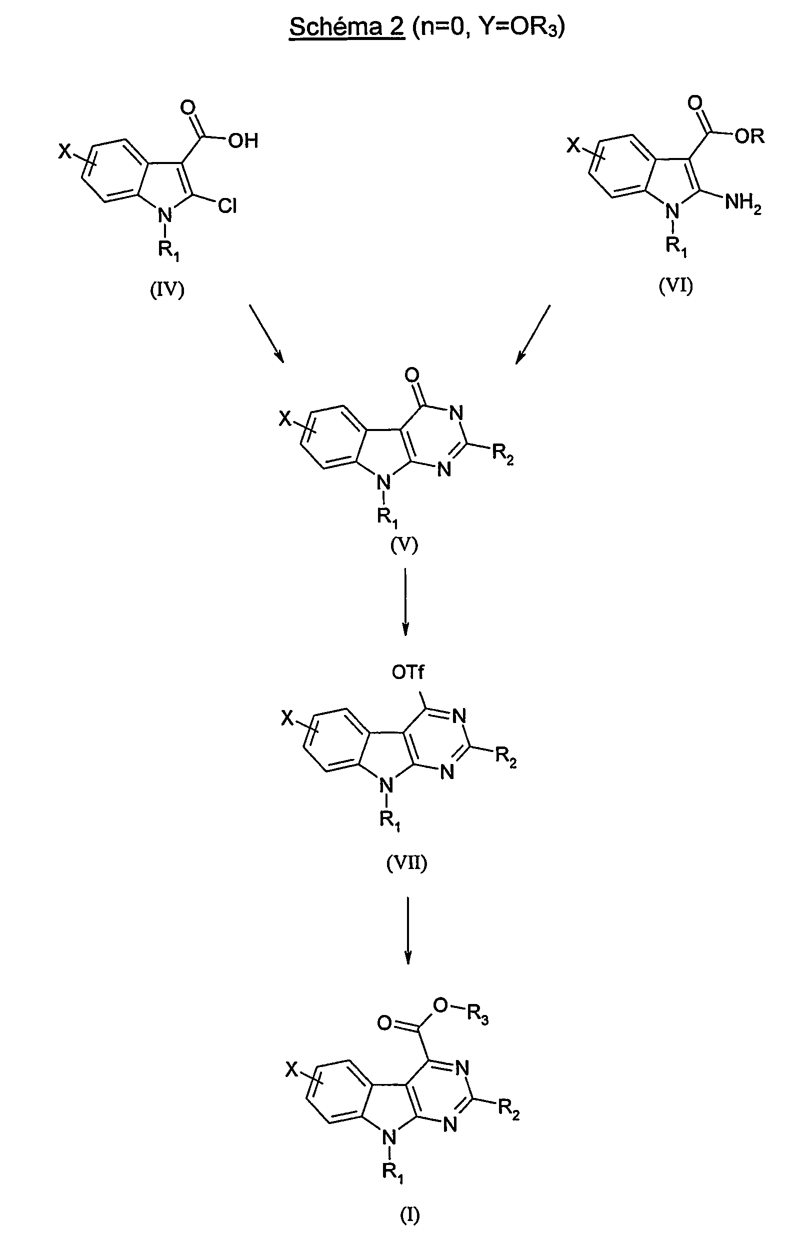

L'ester de formule générale (I), dans laquelle n=0 ; Y=OR₃, R₁=(C₁-C₆)alkyle, X, R₂ et R₃ étant tels que définis ci-dessus, peut être également obtenu à partir de l'amide correspondant de formule générale (I) dans laquelle n=0, Y=NR₄R₅, R₁=(C₁-C₆)alkyle, X, R₂, R₄ et R₅ étant tels que définis ci-dessus, par exemple quand R₄ et R₅ représentent indépendamment l'un de l'autre un méthyle, par action à chaud d'un acide tel que l'acide sulfurique dans un alcool de formule générale R₃OH, dans laquelle R₃ est tel que défini ci-dessus.

Les amides de formule générale (I) dans laquelle n=0, Y=NR₄R₅, X, R₁, R₂, R₄ et R₅ étant tels que définis ci-dessus, peuvent également être obtenus à partir d'un ester de formule générale (I) dans laquelle n=0, Y=OR₃, R₃=(C₁-C₆)alkyle, X, R₁ et R₂ étant tels que définis ci-dessus, par action d'une amine de formule générale HNR₄R₅, dans laquelle R₄ et R₅ sont tels que définis ci-dessus, par exemple en présence d'un dérivé trialkylaluminium dans un solvant tel que le toluène.

Les acides de formule générale (I) dans laquelle n=0, Y=OR₃, R₃=H, X, R₁, R₂, étant tels que définis ci-dessus, peuvent être obtenus par saponification des esters correspondants pour lesquels R₃=(C₁-C₆)alkyle, par exemple en présence d'une base telle que l'hydroxyde de lithium monohydrate dans un solvant tel qu'un mélange de tétrahydrofurane, de méthanol et d'eau.

Les amides de formule générale (I) dans laquelle n=0, Y= NR₄R₅, X, R₁, R₂, R₄ et R₅ étant tels que définis ci-dessus, peuvent également être obtenus par couplage des acides correspondants de formule générale (I) dans laquelle n=0, Y=OR₃, et R₃=H avec une amine de formule générale HNR₄R₅ telle que définie ci-dessus, selon des méthodes connues de l'homme du métier.

### Voie de synthèse pour les acétamides (n=1, Y=NR₄R_{5.})

D'après le schéma 3, un dérivé pyrimido[4,5-*b*]indol-4-one de formule générale (V) telle que définie ci-dessus, est transformé en un 4-halogénopyrimido[4,5-*b*]indole de formule générale (VIII), où X, R₁ et R₂ sont tels que définis ci-dessus et Hal est un atome d'halogène, en employant un agent halogénant, par exemple du chlorure d'oxalyle dans un solvant polaire aprotique tel que de la *N,N-*diméthylformamide, à chaud. Le composé de formule générale (VIII) peut être ensuite condensé avec un malonate de formule générale R' O(CO)CH₂(CO)NR₄R₅, dans laquelle R₄ et R₅ sont tels que définis ci-dessus et R' est un (C₁-C₆)alkyle, en présence d'une base et d'un sel métallique tel qu'un sel de cuivre conduisant à un composé de formule générale (IX) dans laquelle X, R₁, R₂, R₄, R₅ et R' sont tels que définis ci-dessus.

Enfin, le dérivé de structure générale (IX) peut être saponifié et décarboxylé en utilisant par exemple de l'hydroxyde de lithium, dans un mélange de méthanol, d'eau et d'un solvant éthéré, pour conduire à l'amide de formule générale (I) dans laquelle X, R₁, R₂, R₄ et R₅ sont tels que définis ci-dessus.

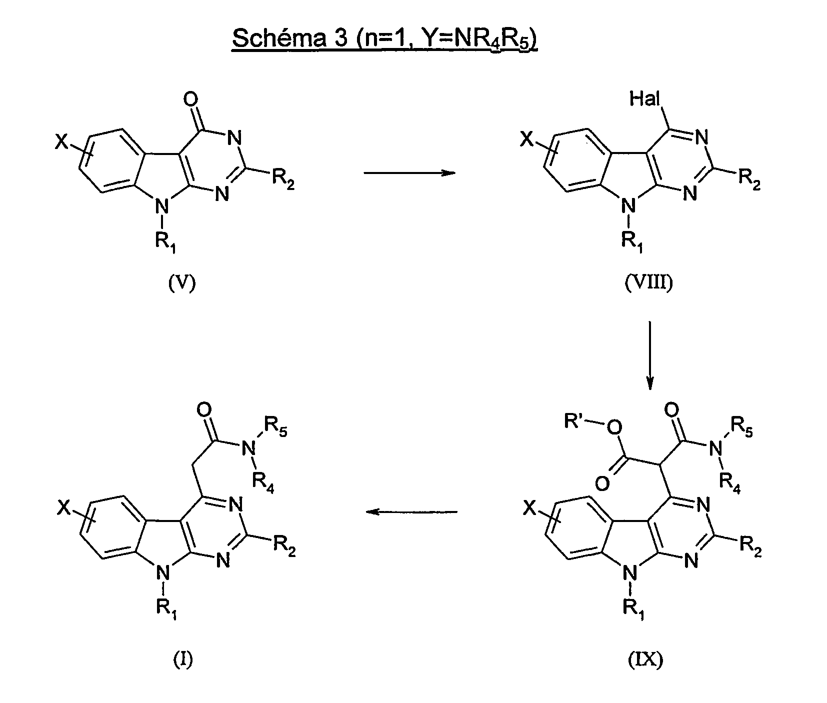

### Voie de synthèse pour les esters acétiques (n=1, Y=OR₃)

Les esters de formule générale (I), dans laquelle n=1, Y=OR₃, R₁=(C₁-C₆)alkyle, R₂ et R₃ étant tels que définis ci-dessus, peuvent être obtenus à partir de l'amide correspondant de formule générale (I) dans laquelle n=1, Y=NR₄R₅, R₁=(C₁-C₆)alkyle, R₂, R₄ et R₅ étant tels que définis ci-dessus, par exemple quand R₄ et R₅ représentent indépendamment l'un de l'autre un méthyle, par action d'un acide tel que l'acide sulfurique dans un solvant alcoolique à chaud.

Les acides de formule générale (I) dans laquelle n=1, Y=OR₃, R₃=H, R₁, R₂, étant tels que définis ci-dessus, peuvent être obtenus à partir des esters correspondants pour lesquels R₃=(C₁-C₆)alkyle, par exemple en présence d'une base telle que l'hydroxyde de lithium monohydrate dans un solvant tel qu'un mélange de tétrahydrofurane, de méthanol et d'eau.

Les composés de départ de formule générale (IIa), (IIb), (IV), (VI), quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les 2-chloroindoles de formule générale (IV), pour lesquels X=H, sont décrits dans la littérature (à titre d'exemple, on peut citer : H.D.H. Showalter et al., J. Med. Chem. (1997), 40, 413 ; P. Kutschy et al., Tetrahedron (2002), 58, 9029). Les 2-chloroindoles de formule générale (IV) pour lesquels X est différent de H peuvent être obtenus à partir des 2-oxindoles de formule générale (IIb) par analogie à des méthodes décrites dans la littérature (à titre d'exemple, on peut citer : L. Sun et al., J. Heterocyclic Chem. (1997), 34, 1399) dans des conditions de réaction de type Vilsmeier, puis par oxydation de l'aldéhyde obtenu.

Les 2-aminoindoles de formule générale (VI), pour lesquels R₁=H, sont décrits ou préparés par des méthodes connues dans la littérature (à titre d'exemple, on peut mentionner : A. Suga et al., JP2002155083, A. Suga et al., WO0174817 ; K.L. Munshi et al., J. Heterocyclic Chem. (1977), 14, 1145 ; C.A. Grob et al., Helv. Chim. Acta, (1961), 44, 1748 ; I.T. Forbes et al., J. Chem. Soc. Perkin Trans. 1, (1992), 275). Les aminoindoles de formule générale (VI), pour lesquels R₁ est différent de H, sont décrits dans le cas où X=H et R₁=Me (R.A. Glennon et al., J. Heterocyclic Chem. (1975), 12, 135). Les aminoindoles de formule générale (VI), pour lesquels R₁ est différent de H, peuvent être obtenus par une réaction d'alkylation à partir des 2-aminoindoles de formule générale (VI) pour lesquels R₁=H par analogie à des méthodes décrites dans la littérature (à titre d'exemple, on peut mentionner : I.T. Forbes et al., J. Chem. Soc. Perkin Trans. 1, (1992), 275 ; R.A. Glennon et al., J. Heterocyclic Chem. (1975), 12, 135).

Les carboxamidines sont connues dans la littérature ou bien peuvent être préparées selon des méthodes qui y sont décrites ou qui sont connues de l'homme du métier, par exemple l'obtention de carboxamidine à partir de nitrile. A titre d'exemple, on peut mentionner la pyrazine-2-carboxamidine décrite dans : J.K. Chakrabarti et al., EP455356.

Les malonates de formule générale R'O(CO)CH₂(CO)NR₄R₅ sont connus dans la littérature et peuvent être préparés selon les méthodes qui y sont décrites. On citera à titre d'exemple : W. Sucrow et al., Chem. Berichte (1968), 101(12), 4230. Les amines de formule générale HNR₄R₅ sont disponibles dans le commerce ou décrites dans la littérature. A titre d'exemple, les amines pour lesquelles R₄ et R₅ forment un cycle pipéridinyle substitué par un groupe azétidinyle, peuvent être préparées par analogie à une méthode décrite dans la littérature (P.C. Ting et al, Bioorg. Med. Chem. Lett. (2001), 11, 491).

Selon un autre de ces aspects, l'invention a également pour objet les composés de formules (III), (VII) et (IX). Ces composés sont utiles comme intermédiaires de synthèse des composés de formule (I).

Les exemples qui suivent illustrent la préparation de quelques composés de l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention. Les microanalyses élémentaires, et les spectres I.R. et R.M.N. confirment les structures des composés obtenus.

### Exemple 1 (Composé N°16)

### 7-chloro-N,N,9-triméthyl-2-pyridin-3-yl-9H-pyrimido[4,5-b]indole-4-carboxamide

### 1.1. 6-chloro-1-méthyl-1,3-dihydro-2H-indol-2-one

On introduit 20 g (0,119 mol) de 6-chloro-1,3-dihydro-2*H*-indol-2-one dans 600 ml d'eau. On ajoute ensuite 17 ml (0,180 mol) de diméthylsulfate et 7,2 g (0,180 mol) de pastilles d'hydroxyde de sodium. On chauffe le mélange à 100°C pendant 20 min. On filtre le mélange à chaud. On conserve l'insoluble contenant l'oxindole de départ. On refroidit le filtrat par bain de glace, puis on le filtre. On récupère un solide blanc que l'on rince avec de l'eau. On ajoute au filtrat l'insoluble obtenu à la première filtration, puis 17 ml (0,180 mol) de diméthylsulfate et 7,2 g (0,180 mol) de pastilles d'hydroxyde de sodium. On porte le mélange à 100°C pendant 20 min. On filtre le mélange à chaud. On conserve l'insoluble. On refroidit le filtrat par bain de glace, puis on le filtre. On récupère un solide blanc que l'on rince avec de l'eau. On recommence encore 3 fois ce cycle. A la fin, on réunit les solides obtenus lors des différentes filtrations et on les sèche sous vide en présence de pentoxyde de phosphore. On purifie le solide par chromatographie sur colonne de gel d'alumine neutre avec un mélange de solvants (éther de pétrole/dichlorométhane : 20/80).

On isole 9,8 g de 6-chloro-1-méthyl-1,3-dihydro-2*H*-indol-2-one sous forme de solide blanc.

P.F. : 116 -117°C

### 1.2. 2-(2,6-dichloro-1-méthyl-1H-indol-3-yl)-N,N-diméthyl-2-oxoacétamide

On introduit sous azote 5,4 ml (63 mmol) de chlorure d'oxalyle dans 100 ml de dichlorométhane. Puis on ajoute lentement une solution de 5,0 g (27,5 mmol) de 6-chloro-1-méthyl-1,3-dihydro-2*H*-indol-2-one, obtenue à l'étape 1.1, dans 30 ml de dichlorométhane. On chauffe le mélange à reflux pendant 4 h. On refroidit le mélange, puis on le concentre sous pression réduite. Sous azote, on dissout le résidu dans 200 ml de dichlorométhane, puis on refroidit le mélange à 0°C. On sature le milieu réactionnel avec de la diméthylamine gazeuse jusqu'à l'obtention d'un pH basique. Après 15 min d'agitation, le mélange est concentré sous pression réduite. Le résidu est purifié par chromatographie sur colonne de gel de silice avec un mélange de solvants (dichlorométhane/méthanol : 100/0 à 99/1). On isole 5,0 g de composé sous forme d'un solide blanc.

P.F. : 146-148°C

### 1.3. 7-chloro-N,N,9-triméthyl-2-pyridin-3-yl-9H-pyrimido[4,5-b]indole-4-carboxamide

On dissout sous azote 0,3 g (13 mmol) de sodium dans 100 ml d'éthanol absolu. On ajoute ensuite 1,0 g (6,34 mmol) de chlorhydrate de pyridine-3-carboxamidine. Après 2 h d'agitation, on concentre le mélange sous pression réduite. On ajoute du dichlorométhane et on filtre. On concentre le filtrat sous pression réduite. On ajoute au résidu 100 ml de xylène, puis 0,34 g (1,13 mmol) de 2-(2,6-dichloro-1-méthyl-1*H*-indol-3-yl)-*N*,*N*-diméthyl-2-oxoacétamide obtenu à l'étape 1.2. On chauffe le mélange à reflux durant 18 h. Puis on le refroidit et on le concentre sous pression réduite. On ajoute du dichlorométhane, de l'eau et une solution aqueuse (1M) d'hydroxyde de sodium. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 80/20 à 0/100). On recristallise le composé obtenu dans un mélange dichlorométhane/acétate d'éthyle, on l'isole par filtration, on le rince avec de l'éther diéthylique et on le sèche sous pression réduite.

On isole 0,18 g de 7-chloro-*N,N*,9-triméthyl-2-pyridin-3-yl-9*H*-pyrimido[4,5-b]indole-4-carboxamide sous forme d'un solide blanc.

P.F.:283-284°C

LC-MS : M+H = 366

H¹ RMN (CDCl₃, 200MHz) : 9,8 (s, 1H) ; 8,9 (m, 1H) ; 8,7 (m, 1H) ; 8,1 (d, 1H) ; 7,3-7,5 (m, 3H) ; 4,0 (s, 3H) ; 3,5 (s, 3H) ; 3,1 (s, 3H).

### Exemple 2 (Composé N°15)

### 7-chloro-N,N,9-triméthyl-2-pyridin-4-yl-9H-pyrimido[4,5-b]indole-4-carboxamide

### 2.1. pyridine-4-carboxamidine

On dissout sous azote 0,44 g (19 mmol) de sodium dans 100 ml de méthanol. On ajoute ensuite 3,0 g (19 mmol) de chlorhydrate de pyridine-4-carboxamidine solubilisée dans 50 ml de méthanol. Après 30 minutes d'agitation, on concentre le mélange sous pression réduite. On ajoute au résidu 150 ml de chloroforme et on chauffe à reflux. On filtre sur célite^{™}. On refroidit le filtrat et on le concentre sous pression réduite.

On obtient 2,25 g de pyridine-4-carboxamidine sous forme d'un solide blanc.

P.F. : 136 -140°C

### 2.2. 7-chloro-N,N,9-triméthyl-2-pyridin-4-yl-9H-pyrimido[4,5-b]indole-4-carboxamide

On chauffe à 130°C pendant 6 h une solution de 2,75 g (9,4 mmol) de 2-(2,6-dichloro-1-méthyl-1*H*-indol-3-yl)-*N*,*N*-diméthyl-2-oxoacétamide, obtenu selon l'étape 1.2. de l'exemple 1, et 4,2 g (34,6 mmol) de pyridine-4-carboxamidine obtenu selon l'étape 2.1. dans 40 ml de *N,N*-diméthylformamide et 10 ml de 1,4-dioxane. Après retour à température ambiante un composé précipite. On l'isole par filtration et on le rince avec de l'acétate d'éthyle. On recristallise le solide dans un mélange acétate d'éthyle/méthanol. Puis on le purifie par chromatographie sur colonne de gel de silice avec un mélange de solvants (dichlorométhane/méthanol: 98/2). On récupère un solide blanc qu'on recristallise dans un mélange acétate d'éthyle/méthanol, qu'on isole par filtration, qu'on rince avec de l'éther diéthylique et qu'on sèche sous pression réduite.

On isole 1,1 g de 7-chloro-*N,N*,9-triméthyl-2-pyridin-4-yl-9*H*-pyrimido[4,5-b]indole-4-carboxamide sous forme d'un solide blanc.

P.F. : 268 -269°C

LC-MS : M+H = 366

H¹ RMN (CDCl₃, 200MHz) : 8,8 (d, 2H) ; 8,5 (d, 2H) ; 8,1 (d, 1H) ; 7,5 (d, 1H) ; 7,3 (dd, 1H) ; 4,0 (s, 3H) ; 3,5 (s, 3H) ; 3,1 (s, 3H).

### Exemple 3 (Composé N°18)

### 7-chloro-N,N,9-triméthyl-2-pyrazin-2-yl-9H-pyrimido[4,5-b]indole-4-carboxamide

On dissout sous azote 0,6 g (26 mmol) de sodium dans 150 ml d'éthanol absolu. On ajoute ensuite 1,2 g (7,6 mmol) de chlorhydrate de pyrazine-2-carboxamidine. Après 1 h 30 d'agitation, l'insoluble résiduel est isolé par filtration et le filtrat est concentré sous pression réduite. On ajoute 150 ml de dichlorométhane et on filtre. On concentre le filtrat sous pression réduite. On ajoute au résidu 100 ml de xylène, puis 0,24 g (0,80 mmol) de 2-(2,6-dichloro-1-méthyl-1*H*-indol-3-yl)-*N,N-*diméthyl-2-oxoacétamide, obtenu selon l'étape 1.2. de l'exemple 1. On chauffe le mélange à reflux durant 18 h. On le refroidit ensuite et on le concentre sous pression réduite. On ajoute du dichlorométhane, de l'eau et une solution aqueuse (1 M) d'hydroxyde de sodium. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 80/20 à 0/100, puis acétate d'éthyle/méthanol : 100/0 à 95/5). On recristallise le composé obtenu dans un mélange acétate d'éthyle/méthanol, on l'isole par filtration, on le rince avec de l'éther diéthylique et on le sèche sous pression réduite.

On isole 0,070 g de 7-chloro-*N,N*,9-triméthyl-2-pyrazin-2-yl-9*H*-pyrimido[4,5-b]-indole-4-carboxamide sous forme d'un solide blanc.

P.F. : 272 - 273°C

LC-MS : M+H = 367

H¹ RMN (CDCl₃, 200MHz) : 9,9 (s, 1H) ; 8,9 (d, 1H) ; 8,7 (d, 1H) ; 8,1 (d, 1H) ; 7,6 (d, 1H) ; 7,4 (dd, 1H) ; 4,1 (s, 3H) ; 3,4 (s, 3H) ; 3,1 (s, 3H).

### Exemple 4 (Composé N°14)

### 7-chloro-9-méthyl-2-pyridin-4-yl-4-(pyrrolidin-1-ylcarbonyl)-9H-pyrimido[4,5-b]indole

### 4.1. 1-(2,6-dichloro-1-méthyl-1H-indol-3-yl)-2-oxo-2-pyrrolidin-1-yléthanone

On introduit sous azote 5,0 ml (58,2 mmol) de chlorure d'oxalyle dans 120 ml de dichlorométhane. On ajoute ensuite lentement une solution de 3,0 g (16,5 mmol) de 6-chloro-1-méthyl-1,3-dihydro-2*H*-indol-2-one, obtenue à l'étape 1.1 de l'exemple 1, dans 30 ml de dichlorométhane. On chauffe le mélange à reflux pendant 4 h. On refroidit le mélange, puis on le concentre sous pression réduite. Sous azote, on dissout le résidu dans 150 ml de dichlorométhane et on refroidit le mélange à 0°C. On ajoute ensuite lentement 2,8 ml (33,8 mmol) de pyrrolidine. Après 15 min d'agitation environ, on ajoute une solution aqueuse 1 M d'acide chlorhydrique jusqu'à un pH de 3-4. On décante la phase organique, on la lave avec de l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 95/5 à 70/30). On recristallise le solide obtenu dans un mélange dichlorométhane/acétate d'éthyle, on l'isole par filtration, on le rince avec de l'éther diéthylique et on le sèche sous pression réduite.

On isole 3,3 g de 1-(2,6-dichloro-1-méthyl-1*H*-indol-3-yl)-2-oxo-2-pyrrolidin-1-yléthanone sous forme d'un solide blanc.

P.F. : 174 -175°C

### 4.2. 7-chloro-9-méthyl-2-pyridin-4-yl-4-(pyrrolidin-1-ylcarbonyl)-9H-pyrimido[4,5-b]indole

On chauffe à reflux pendant 5 h une solution de 1,0 g (3,07 mmol) de 1-(2,6-dichloro-1-méthyl-1*H*-indol-3-yl)-2-oxo-2-pyrrolidin-1-yléthanone, obtenu à l'étape 4.1, et 1,3 g (10,6 mmol) de pyridine-4-carboxamidine, obtenu selon l'étape 2.1 de l'exemple 2, dans 60 ml de xylène. Après retour à température ambiante, on concentre le mélange sous pression réduite. On ajoute 200 ml de dichlorométhane, on lave la phase organique avec une solution aqueuse saturée en hydrogénocarbonate de sodium, puis avec de l'eau. On sèche la phase organique sur sulfate de sodium, on la filtre et on la concentre sous pression réduite. On purifie le résidu par chromatographie sur colonne de gel de silice avec un mélange de solvants (dichlorométhane/acétate d'éthyle : 90/10 à 0/100). On récupère un solide blanc qu'on recristallise dans un mélange dichlorométhane/acétate d'éthyle, on l'isole par filtration, on le rince avec de l'éther diéthylique et on le sèche sous pression réduite.

On isole 0,40 g de 7-chloro-9-méthyl-2-pyridin-4-yl-4-(pyrrolidin-1-ylcarbonyl)-9*H* pyrimido[4,5-b]indole sous forme d'un solide blanc.

P.F. : 256 - 258°C

LC-MS : M+H = 392

H¹ RMN (CDCl₃, 300MHz) : 8,8 (m, 2H) ; 8,5 (m, 2H) ; 8,4 (d, 1H) ; 7,5 (d, 1H) ; 7,4 (d, 1H) ; 4,0 (s, 3H) ; 3,9 (t, 2H) ; 3,8 (t, 2H) ; 2,1-1,9 (m, 4H).

### Exemple 5 (Composé N°5)

### 7-chloro-N,N-diméthyl-2-phényl-9H-pyrimido[4,5-b]indole-4-carboxamide

### 5.1. 2-(2,6-dichloro-1H-indol-3-yl)-N,N-diméthyl-2-oxoacétamide

Sous azote, on introduit 8 ml (91,7 mmol) de chlorure d'oxalyle dans 150 ml de dichlorométhane. On ajoute ensuite par petites portions 5,0 g (29,8 mmol) de 6-chloro-1,3-dihydro-2*H*-indol-2-one. On agite le mélange 2 h à température ambiante, puis 4 h à reflux. On refroidit le mélange, puis on le concentre sous pression réduite. Sous azote, on dissout le résidu dans 150 ml de dichlorométhane et on refroidit le mélange à 0°C. On sature le milieu réactionnel avec de la diméthylamine gazeuse jusqu'à l'obtention d'un pH basique. Après 15 min d'agitation, on ajoute une solution aqueuse 1 M d'acide chlorhydrique jusqu'à un pH de 2-3. On ajoute ensuite 300 ml de dichlorométhane et 100 ml d'eau. On décante la phase organique, on la lave avec de l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 90/10 à 50/50). On rince le solide obtenu avec de l'éther diéthylique et on le sèche sous pression réduite.

On isole 3,2 g de 2-(2,6-dichloro-1*H*-indol-3-yl)-*N,N*-diméthyl-2-oxoacétamide sous forme d'un solide blanc.

P.F. : 223 - 224°C

### 5.2. 7-chloro-N,N-diméthyl-2-phényl-9H-pyrimido[4,5-b]indole-4-carboxamide

On dissout sous azote 0,2 g (8,3 mmol) de sodium dans 100 ml d'éthanol absolu. On ajoute ensuite 1,0 g (6,38 mmol) de chlorhydrate de benzamidine. Après 30 minutes d'agitation, on concentre le mélange sous pression réduite. On ajoute du dichlorométhane et on filtre. On concentre le filtrat sous pression réduite. On ajoute au résidu 120 ml de toluène, puis 0,30 g (1,2 mmol) de 2-(2,6-dichloro-1*H-*indol-3-yl)-*N,N*-diméthyl-2-oxoacétamide, obtenu à l'étape 5.1. de l'exemple 5. On chauffe le mélange à reflux durant 18 h. Puis on le refroidit et on le concentre sous pression réduite. On ajoute du dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 95/5 à 50/50). On recristallise le composé obtenu dans un mélange dichlorométhane/acétate d'éthyle, on l'isole par filtration, on le rince avec de l'éther diéthylique et on le sèche sous pression réduite.

On isole 0,09 g de 7-chloro-*N,N* diméthyl-2-phényl-9*H*-pyrimido[4,5-b]indole-4-carboxamide sous forme d'un solide blanc.

P.F. : 232 - 233°C

LC-MS : M+H = 351

H¹ RMN (DMSOd₆, 200MHz) : 12,7 (s, 1H) ; 8,5 (m, 2H) ; 7,9 (d, 1H) ; 7,5-7,6 (m, 4H) ; 7,4 (dd, 1H) ; 3,2 (s, 3H) ; 3,0 (s, 3H).

### Exemple 6 (Composé N°4)

### 6-chloro-N,N,9-triméthyl-2-phényl-9H-pyrimido[4,5-b]indole-4-carboxamide

### 6.1. 5-chloro-1-méthyl-1,3-dihydro-2H-indol-2-one

On introduit 13,3 g (0,795 mol) de 5-chloro-1,3-dihydro-2*H*-indol-2-one dans 200 ml d'eau. On ajoute ensuite 120 ml d'une solution 1 M d'hydroxyde de sodium et 11,3 ml (0,120 mol) de diméthylsulfate. On chauffe le mélange à 120°C pendant 30 min. On laisse refroidir, puis on ajoute 4,81 g (120 mmol) d'hydroxyde de sodium et 11,3 ml (0,120 mol) de diméthylsulfate. On chauffe le mélange à 120°C pendant 30 min. On laisse refroidir et on répète l'opération encore une fois. On filtre ensuite le mélange réactionnel et on rince le précipité avec de l'eau et on le sèche sous vide en présence de pentoxyde de phosphore. On ajoute au filtrat 250 ml de dichlorométhane. On décante la phase organique, on la lave à l'eau, on la sèche sur sulfate de sodium, on la filtre et on la concentre sous pression réduite. On ajoute au résidu obtenu le précipité et on purifie l'ensemble par chromatographie sur colonne de gel d'alumine neutre avec un mélange de solvants (dichlorométhane/acétate d'éthyle : 98/2 à 50/50).

On isole 3,9 g de 5-chloro-1-méthyl-1,3-dihydro-2*H*-indol-2-one sous forme d'un solide blanc.

P.F. : 118-119°C

### 6.2. 2-(2,5-dichloro-1-méthyl-1H-indol-3-yl)-N,N-diméthyl-2-oxoacétamide

On introduit sous azote 8,1 ml (92,8 mmol) de chlorure d'oxalyle dans 150 ml de dichlorométhane. On ajoute ensuite par petites portions 3,9 g (30,7 mmol) de 5-chloro-1-méthyl-1,3-dihydro-2*H*-indol-2-one, obtenu à l'étape 6.1. de l'exemple 6. On chauffe le mélange à reflux pendant 4 h. On refroidit le mélange, puis on le concentre sous pression réduite. On dissout le résidu sous azote dans 120 ml de dichlorométhane et on refroidit le mélange à 0°C. On sature le milieu réactionnel avec de la diméthylamine gazeuse jusqu'à l'obtention d'un pH basique. Après 15 min d'agitation, on concentre le mélange sous pression réduite. On ajoute 250 ml de dichlorométhane, 100 ml d'eau et une solution aqueuse 1M d'acide chlorhydrique jusqu'à un pH de 3-4. On décante la phase organique, on la lave avec de l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 95/5 à 50/50).

On isole 1,45 g de 2-(2,5-dichloro-1-méthyl-1*H*-indol-3-yl)-*N*,*N*-diméthyl-2-oxoacétamide sous forme d'un solide blanc.

P.F. : 205 - 206°C

### 6.3. 6-chloro-N,N,9-triméthyl-2-phényl-9H-pyrimido[4,5-b]indole-4-carboxamide

On dissout sous azote 0,34 g (14,8 mmol) de sodium dans 120 ml d'éthanol absolu. On ajoute ensuite 2,0 g (12,8 mmol) de chlorhydrate de benzamidine. Après 30 minutes d'agitation, on concentre le mélange sous pression réduite. On ajoute du dichlorométhane et on filtre. On concentre le filtrat sous pression réduite. On ajoute au résidu 60 ml de xylène, puis 0,90 g (3 mmol) de 2-(2,5-dichloro-1-méthyl-1*H*-indol-3-yl)-*N*,*N*-diméthyl-2-oxoacétamide, obtenu à l'étape 6.2. de l'exemple 6. On chauffe le mélange à reflux durant 3 h. Puis on le refroidit et on le concentre sous pression réduite. On ajoute du dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 95/5 à 50/50). On recristallise le composé obtenu dans un mélange dichlorométhane/acétate d'éthyle, on l'isole par filtration, on le rince avec de l'éther diéthylique et on le sèche sous pression réduite.

On isole 0,53 g de 6-chloro-*N,N*,9-triméthyl-2-phényl-9*H*-pyrimido[4,5-b]indole-4-carboxamide sous forme d'un solide blanc.

P.F. : 222 - 224°C

LC-MS : M+H = 365

H¹ RMN (CDCl₃, 200MHz) : 8,7 (m, 2H) ; 8,1 (d, 1H) ; 7,6-7,5 (m, 4H) ; 7,4 (d, 1H) ; 4,1 (s, 3H) ; 3,4 (s, 3H) ; 3,2 (s, 3H).

### Exemple 7 (Composé N°23)

### N,N-diméthyl-2-phényl-9H-pyrimido[4,5-b]indole-4-carboxamide

### 7.1. 2-(2-chloro-1H-indol-3-yl)-N,N-diméthyl-2-oxoacétamide

On introduit sous azote 7,9 ml (90,5 mmol) de chlorure d'oxalyle dans 150 ml de dichlorométhane. On ajoute ensuite par petites portions 6,0 g (45 mmol) de 1,3-dihydro-2*H*-indol-2-one. On agite le mélange à température ambiante pendant 4 h. On filtre et on solubilise ensuite le solide dans 150 ml de dichlorométhane. On refroidit le mélange à 0°C. On sature le milieu réactionnel avec de la diméthylamine gazeuse jusqu'à l'obtention d'un pH basique. Après 5 min d'agitation, on concentre le mélange sous pression réduite. On purifie le résidu par chromatographie sur colonne de gel de silice avec un mélange de solvants (dichlorométhane/acétate d'éthyle : 95/5 à 50/50).

On isole 2,46 g de 2-(2-chloro-1*H*-indol-3-yl)-*N,N*-diméthyl-2-oxoacétamide sous forme d'un solide blanc.

P.F. : 197 - 198°C

### 7.2. N,N-diméthyl-2-phényl-9H-pyrimido[4,5-b]indole-4-carboxamide

On dissout sous azote 0,40 g (17,4 mmol) de sodium dans 100 ml de méthanol. On ajoute ensuite 2,72 g (17,4 mmol) de chlorhydrate de benzamidine. Après 30 minutes d'agitation, on concentre le mélange sous pression réduite. On ajoute du dichlorométhane et on filtre. On concentre le filtrat sous pression réduite. On ajoute au résidu 100 ml de xylène, puis 1,52 g (6,1 mmol) de 2-(2-chloro-1*H-*indol-3-yl)-*N*,*N*-diméthyl-2-oxoacétamide obtenu à l'étape 7.1. de l'exemple 7. On chauffe le mélange à reflux durant 5 h. Puis on le refroidit et on le concentre sous pression réduite. On ajoute du dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 90/10 à 70/30). On concentre, sous pression réduite, les fractions contenant le composé jusqu'à l'apparition d'un précipité. On l'isole par filtration, on le rince avec de l'éther diéthylique et on le sèche sous pression réduite.

On isole 1,36 g de *N,N*-diméthyl-2-phényl-9*H*-pyrimido[4,5-b]indole-4-carboxamide sous forme de solide blanc.

P.F. : 218 - 219°C

MS : M+H=317

H¹ RMN (DMSOd₆, 200MHz) : 12,6 (s, 1H) ; 8,5 (m, 2H) ; 7,9 (d, 1H) ; 7,6-7,5 (m, 5H) ; 7,3 (m, 1H) ; 3,2 (s, 3H) ; 2,9 (s, 3H).

### Exemple 8 (Composé N°1)

### N,N,9-triméthyl-2-phényl-9H-pyrimido[4,5-b]indole-4-carboxamide

On chauffe à reflux 6 h, sous azote, 0,40 g (1,3 mmol) de *N,N*-diméthyl-2-phényl-9*H*-pyrimido[4,5-b]indole-4-carboxamide obtenu à l'étape 7.2. de l'exemple 7 et 1 ml (7,5 mmol) de 1,1-diméthoxytriméthylamine dans 120 ml de toluène. On refroidit ensuite à température ambiante et on concentre sous pression réduite. On purifie le résidu par chromatographie sur colonne de gel de silice avec un mélange de solvants (dichlorométhane/acétate d'éthyle : 100/0 à 50/50). On réunit et on concentre les fractions contenant le produit sous pression réduite. On recristallise le composé dans de l'acétate d'éthyle, on l'isole par filtration, on le rince avec de l'éther diéthylique et on le sèche sous pression réduite.

On isole 0,33 g de *N,N*,9-triméthyl-2-phényl-9*H*-pyrimido[4,5-b]indole-4-carboxamide sous forme d'un solide blanc.

P.F. : 193-195°C

LC-MS : M+H = 331

H¹ RMN (CDCl₃, 200MHz) : 8,7 (m, 2H) ; 8,1 (d, 1H) ; 7,6-7,5 (m, 5H) ; 7,4 (m, 1H) ; 4,1 (s, 3H) ; 3,4 (s, 3H) ; 3,1 (s, 3H).

### Exemple 9 (Composé N°3)

### 7-fluoro-N,N,9-triméthyl-2-phényl-9H-pyrimido[4,5-b]indole-4-carboxamide

### 9.1. 6-fluoro-1-méthyl-1,3-dihydro-2H-indol-2-one

On introduit 10,0g (66,5 mmol) de 6-fluoro-1,3-dihydro-2*H*-indol-2-one dans 200 ml d'eau. On ajoute ensuite 120 ml d'une solution 1 M d'hydroxyde de sodium et 11,3 ml (120 mmol) de diméthylsulfate. On chauffe le mélange à 120°C pendant 40 min. Après retour à température ambiante, on ajoute 150 ml de dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel d'alumine neutre avec un mélange de solvants (dichlorométhane/acétate d'éthyle : 98/2 à 80/20). On rince le composé obtenu avec de l'éther diéthylique et on le sèche sous pression réduite.

On isole 5,1 g de 6-fluoro-1-méthyl-1,3-dihydro-2*H*-indol-2-one sous forme d'un composé jaunâtre.

P.F. : 100 - 101 °C

### 9.2. 2-(2-chloro-6-fluoro-1-méthyl-1H-indol-3-yl)-N,N diméthyl-2-oxoacétamide

On introduit sous azote 5,0 ml (58,2 mmol) de chlorure d'oxalyle dans 120 ml de dichlorométhane. On ajoute ensuite lentement une solution de 4,2 g (25,4 mmol) de 6-fluoro-1-méthyl-1,3-dihydro-2*H*-indol-2-one, obtenue à l'étape 9.1 de l'exemple 9, par petites portions. On chauffe le mélange à reflux pendant 4 h. On refroidit le mélange, puis on le concentre sous pression réduite. Sous azote, on dissout le résidu dans 120 ml de dichlorométhane et on refroidit le mélange à 0°C. On sature le milieu réactionnel avec de la diméthylamine gazeuse jusqu'à l'obtention d'un pH basique. Après 15 min d'agitation, on ajoute une solution aqueuse d'acide chlorhydrique 1 M jusqu'à un pH de 3-4. On ajoute ensuite 100 ml de dichlorométhane. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 95/5 à 50/50). On concentre sous pression réduite les fractions contenant le composé jusqu'à l'apparition d'un précipité. On le recueille par filtration et on le rince avec de l'éther diéthylique et on le sèche sous pression réduite.

On isole 3,5 g de 2-(2-chloro-6-fluoro-1-méthyl-1*H*-indol-3-yl)-*N,N*-diméthyl-2-oxoacétamide sous forme d'un composé blanc.

P.F. : 175 - 177°C

### 9.3. 7-fluoro-N,N,9-triméthyl-2-phényl-9H-pyrimido[4,5-b]indole-4-carboxamide

On dissout sous azote 0,28 g (12,17 mmol) de sodium dans 120 ml de méthanol. On ajoute ensuite 1,89 g (12,07 mmol) de chlorhydrate de benzamidine. Après 30 minutes d'agitation, on concentre le mélange sous pression réduite. On ajoute du dichlorométhane et on filtre. On concentre le filtrat sous pression réduite. On ajoute au résidu 50 ml de xylène, puis 0,80 g (2,8 mmol) de 2-(2-chloro-6-fluoro-1-méthyl-1*H*-indol-3-yl)-*N,N*-diméthyl-2-oxoacétamide, obtenu à l'étape 9.2. de l'exemple 9. On chauffe le mélange à reflux durant 6 h. Puis on le refroidit et on le concentre sous pression réduite. On ajoute du dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 98/2 à 90/10). On concentre les fractions contenant le composé sous pression réduite jusqu'à l'apparition d'un précipité. On le recueille par filtration et on le rince avec de l'éther diéthylique. On recristallise le composé obtenu dans un mélange dichlorométhane/acétate d'éthyle, on l'isole par filtration, on le rince avec de l'éther diéthylique et on le sèche sous pression réduite.

On isole 0,40 g de 7-fluoro-*N,N*,9-triméthyl-2-phényl-9*H*-pyrimido[4,5-b]indole-4-carboxamide sous forme d'un solide blanc.

P.F. : 229 - 31 °C

LC-MS : M+H = 349

H¹ RMN (DMSOd₆, 200MHz) : 8,6 (m, 2H) ; 7,9 (dd, 1H) ; 7,7 (dd, 1H) ; 7,5 (m, 3H) ; 7,2 (m, 1H) ; 4,0 (s, 3H) ; 3,2 (s, 3H) ; 3,0 (s, 3H).

### Exemple 10 (Composé N°24)

### 7-chloro-9-méthyl-2-phényl-9H-pyrimido[4,5-b]indole-4-carboxylate d'éthyle

On chauffe à reflux pendant 48 h une solution de 1,6 g (4,4 mmol) de 7-chloro-*N,N*,9-triméthyl-2-phényl-9*H*-pyrimido[4,5-b]indole-4-carboxamide, obtenu selon l'exemple 16 dans 80 ml d'éthanol absolu et 2,5 ml d'acide sulfurique. On refroidit le mélange à température ambiante et on concentre partiellement sous pression réduite. On ajoute ensuite de la glace pilée et du dichlorométhane. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 100/0 à 80/20). On concentre les fractions contenant le composé sous pression réduite. On reprend le résidu par du cyclohexane, on le recueille par filtration et on le rince avec du pentane.

On isole 0,46 g de 7-chloro-9-méthyl-2-phényl-9*H*-pyrimido[4,5-b]indole-4-carboxylate d'éthyle sous forme d'un composé blanc.

P.F. : 139 - 140°C

MS : M+H = 366

H¹ RMN (CDCl₃, 200MHz) : 8,8 (d, 1H) ; 8,6 (m, 2H) ; 7,6-7,5 (m, 4H) ; 7,3 (dd, 1H) ; 4,7 (q, 2H) ; 4,0 (s, 3H) ; 1,6 (t, 3H).

### Exemple 11 (Composé N°11)

### 7-chloro-9-méthyl-4-[(4-méthylpiperazin-1-yl)carbonyl]-2-phényl-9H-pyrimido[4,5-b]indole

On ajoute à 30 ml de toluène 4 ml (8 mmol) d'une solution de triméthylaluminium (2M dans le toluène) sous argon. On refroidit la solution à 0°C, puis on ajoute par portions 0,80 g (8 mmol) de 4-méthylpipérazine. On maintient l'agitation pendant 1 h à température ambiante. On ajoute ensuite 0,46 g (1,20 mmol) de 7-chloro-9-méthyl-2-phényl-9*H*-pyrimido[4,5-b]indole-4-carboxylate d'éthyle obtenu à l'exemple 10. On chauffe le milieu réactionnel à reflux durant 3 h. On refroidit la solution vers 0°C, puis on additionne de l'eau goutte à goutte. On ajoute ensuite du dichlorométhane, puis une solution de soude concentrée. On décante la phase organique, on la lave à l'eau, on la sèche sur sulfate de sodium, puis on la filtre et on la concentre sous pression réduite. On purifie le résidu par chromatographie sur colonne de gel de silice avec un mélange de solvants (dichlorométhane/acétate d'éthyle : 50/50, puis acétate d'éthyle pur, puis acétate d'éthyle/méthanol : 95/5). On concentre les fractions contenant le composé sous pression réduite. On isole 0,54 g d'un composé blanc. On ajoute à ce composé du dichlorométhane et 13 ml d'une solution 0,1 N d'acide chlorhydrique dans le propan-2-ol. On concentre la solution sous pression réduite. On recristallise le résidu dans de l'éthanol et on le rince avec de l'éther diéthylique. On ajoute au composé obtenu du dichlorométhane puis une solution de soude aqueuse 1 M. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/méthanol : 98/2 à 90/10). On concentre les fractions contenant le composé sous pression réduite. On recristallise le composé obtenu dans un mélange dichlorométhane/acétate d'éthyle, on le recueille par filtration, on le rince avec de l'éther diéthylique et on le sèche sous pression réduite.

On isole 0,43 g de 7-chloro-9-méthyl-4-[(4-méthylpiperazin-1-yl)carbonyl]-2-phényl-9*H*-pyrimido[4,5-b]indole sous forme d'un solide blanc.

P.F. : 236-238°C

LC-MS : M+H = 420

H¹ RMN (CDCl₃, 200MHz) : 8,8 (m, 2H) ; 8,1 (d, 1H) ; 7,6-7,5 (m, 4H) ; 7,3 (dd, 1H) ; 4,1 (m, 2H) ; 4,0 (s, 3H) ; 3,6 (m, 2H) ; 2,7 (m, 2H) ; 2,5 (m, 2H) ; 2,4 (s, 3H).

### Exemple 12 (Composé N°21)

### 2-(7-chloro-9-méthyl-2-pyridin-4-yl-9H-pyrimido[4,5-b]indol-4-yl)-N,N-diméthyl-acétamide

### 12.1. 2-amino-6-chloro-1-méthyl-1H-indole-3-carboxylate d'éthyle

On introduit sous azote 2,5 g (62,5 mmol) d'hydrure de sodium (à 60% dans de l'huile minérale) dans 200 ml de tétrahydrofurane. On refroidit à 0°C et on ajoute 14,89 g (62,38 mmol) de 2-amino-6-chloro-1*H-*indole-3-carboxylate d'éthyle par portions. Après 1 h d'agitation à 0°C, on ajoute 3,9 ml (62,6 mmol) d'iodométhane et on agite 12 h à température ambiante. On ajoute 3 ml d'éthanol absolu et on concentre le mélange sous pression réduite. On purifie le résidu par chromatographie sur colonne de gel de silice avec un mélange de solvants (dichlorométhane/acétate d'éthyle : 98/2 à 80/20). On concentre les fractions contenant le composé sous pression réduite.

On isole 5,9 g de 2-amino-6-chloro-1-méthyl-1*H*-indole-3-carboxylate d'éthyle sous forme d'un solide.

P.F. : 155-157°C

### 12.2. 7-chloro-9-méthyl-2-pyridin-4-yl-3,9-dihydro-4H-pyrimido[4,5-b]indole-4-one

On introduit sous azote 0,32 g (8 mmol) d'hydrure de sodium (à 60 % dans de l'huile minérale) dans 100 ml de tétrahydrofurane. On refroidit à 0°C et on ajoute 2,0 g (7,91 mmol) de 2-amino-6-chloro-1-méthyl-1*H*-indole-3-carboxylate d'éthyle, obtenu à l'étape 12.1. de l'exemple 12. Après 40 min d'agitation à 0°C, on enlève le bain réfrigéré et on ajoute 2 g (19,2 mmol) de 4-cyanopyridine. On agite ensuite le mélange à 80°C durant 18 h. On refroidit à température ambiante puis on concentre le mélange sous pression réduite. On ajoute au résidu du dichlorométhane, de l'eau et une solution aqueuse 1 M d'hydroxyde de sodium. On agite le mélange 2 h. On recueille par filtration un insoluble, on le rince à l'eau et on le sèche en étuve sous pression réduite en présence de pentoxyde de phosphore.

On isole 1,55 g de 7-chloro-9-méthyl-2-pyridin-4-yl-3,9-dihydro-4*H*-pyrimido[4,5-b]indole-4-one sous forme d'un solide.

P.F. : > 300°C

### 12.3. 4,7-dichloro-9-méthyl-2-pyridin-4-yl-9H-pyrimido[4,5-b]indole

Sous azote et à 0°C, on ajoute lentement 14 ml (160 mmol) de chlorure d'oxalyle à 120 ml de *N,N*-diméthylformamide. Après 30 min d'agitation, on ajoute à 0°C 1,55 g (5 mmol) de 7-chloro-9-méthyl-2-pyridin-4-yl-3,9-dihydro-4*H*-pyrimido[4,5-b]indole-4-one, obtenu à l'étape 12.2. de l'exemple 12. On enlève le bain réfrigéré et on chauffe le mélange à 80°C pendant 18 h. On refroidit à température ambiante et on verse le mélange sur de la glace pilée. On ajoute ensuite du dichlorométhane et une solution aqueuse d'hydroxyde de sodium à 30%. Après agitation, on décante la phase organique, on la lave à l'eau, on la sèche sur sulfate de sodium, on la filtre et on la concentre sous pression réduite. On solubilise partiellement le résidu dans du dichlorométhane. On sépare l'insoluble par filtration et on purifie le filtrat par chromatographie sur colonne de gel de silice avec un mélange de solvants (dichlorométhane/acétate d'éthyle : 80/20 à 50/50). On réunit les fractions contenant le composé avec l'insoluble, puis on les concentre partiellement sous pression réduite. On recueille un précipité par filtration que l'on rince avec de l'éther diéthylique.

On isole 0,83 g de 4,7-dichloro-9-méthyl-2-pyridin-4-yl-9*H*-pyrimido[4,5-b]indole sous forme d'un solide blanc.

P.F. : 272 - 274°C

### 12.4. 2-(7-chloro-9-méthyl-2-pyridin-4-yl-9H-pyrimido[4,5-b]indol-4-yl)-3-(diméthylamino)-3-oxopropanoate d'éthyle

On introduit sous azote 0,55 g (13,7 mmol) d'hydrure de sodium (à 60% dans de l'huile minérale) dans 150 ml de 1,4-dioxane. On refroidit à 0°C et on ajoute lentement 2,2 g (13,8 mmol) de 3-(diméthylamino)-3-oxopropanoate d'éthyle. Après 40 min d'agitation à 0°C, on ajoute 0,83 g (2,52 mmol) de 4,7-dichloro-9-méthyl-2-pyridin-4-yl-9*H*-pyrimido[4,5-b]indole, obtenu à l'étape 12.3 de l'exemple 12, puis on enlève le bain réfrigéré. On additionne ensuite 2,5 g (13,1 mmol) d'iodure cuivreux et on chauffe le mélange pendant 6 h à reflux. On refroidit à température ambiante et on concentre sous pression réduite. On ajoute ensuite du dichlorométhane, de l'eau et une solution aqueuse saturée en hydrogénocarbonate de sodium. Après agitation, on décante la phase organique, on la lave à l'eau, on la sèche sur sulfate de sodium, on la filtre et on la concentre sous pression réduite. On purifie le résidu par chromatographie sur colonne de gel de silice avec un mélange de solvants (acétate d'éthyle/méthanol : 100/0 à 90/10, puis ajout de 2 % d'ammoniaque). On concentre partiellement les fractions contenant le composé sous pression réduite. On isole un précipité par filtration, on le rince avec de l'éther diéthylique, et on le sèche sous pression réduite.

On isole 0,79 g de 2-(7-chloro-9-méthyl-2-pyridin-4-yl-9*H*-pyrimido[4,5-b]indol-4-yl)-3-(diméthylamino)-3-oxopropanoate d'éthyle sous forme d'un solide jaunâtre. P.F. : 224 -226°C

### 12.5. 2-(7-chloro-9-méthyl-2-pyridin-4-yl-9H-pyrimido[4,5-b]indol-4-yl)-N,N-diméthylacétamide

On agite pendant 4 h à 50°C une solution de 0,79 g (1,85 mmol) de 2-(7-chloro-9-méthyl-2-pyridin-4-yl-9*H*-pyrimido[4,5-b]indol-4-yl)-3-(diméthylamino)-3-oxopropanoate d'éthyle obtenu à l'étape 12.4, avec 1,4 g (33,3 mmol) d'hydroxyde de lithium monohydrate dans un mélange de 10 ml d'eau, 50 ml de tétrahydrofurane et 50 ml de méthanol. On refroidit le mélange à température ambiante et on le concentre sous pression réduite. On ajoute lentement une solution d'acide chlorhydrique à 35 % dans l'eau et on agite 5 min. On ajoute ensuite lentement une solution d'hydroxyde de sodium aqueuse à 30 % jusqu'à un pH basique. On ajoute du dichlorométhane. On décante la phase organique. On extrait la phase aqueuse avec du dichlorométhane. On réunit les phases organiques, on les lave à l'eau, on les sèche sur sulfate de sodium, on les filtre et on les concentre sous pression réduite. On purifie le résidu par chromatographie sur colonne de gel de silice avec un mélange de solvants (acétate d'éthyle/méthanol : 100/0 à 90/10). On concentre les fractions contenant le composé sous pression réduite. On recristallise le solide dans un mélange acétate d'éthyle/méthanol, et on le rince avec de l'éther diéthylique.

On isole 0,19 g de 2-(7-chloro-9-méthyl-2-pyridin-4-yl-9*H*-pyrimido[4,5-b]indol-4-yl)-*N,N*-diméthylacétamide sous forme d'un solide blanc.

P.F. : 231 - 232°C

LC-MS : M+H = 380

H¹ RMN (DMSOd₆, 200MHz) : 8,8 (m, 2H) ; 8,4 (m, 2H) ; 8,2 (d, 1H) ; 8,0 (d, 1H) ; 7,4 (dd, 1H) ; 4,5 (s, 2H) ; 4,0 (s, 3H) ; 3,2 (s, 3H) ; 2,9 (s, 3H).

### Exemple 13 (Composé N°19)

### 2-(7-chloro-9-méthyl-2-phényl-9H-pyrimido[4,5-b]indol-4-yl)-N,N-diméthyl-acétamide

### 13.1. 2,6-dichloro-1H-indole-3-carbaldéhyde

Sous azote on ajoute à 0°C goutte à goutte 27 ml (292 mmol) d'oxychlorure de phosphore à 28 ml (366 mmol) de *N,N*-diméthylformamide dans 190 ml de dichlorométhane. On agite pendant 2 h à température ambiante. On refroidit à 0°C et on ajoute par petites portions 24,5 g (146 mmol) de 6-chloro-1,3-dihydro-2*H*-indol-2-one. On agite pendant 4 h à température ambiante. On refroidit à 0°C et on ajoute 12,7 ml (146 mmol) de chlorure d'oxalyle. On agite ensuite pendant 12 h à température ambiante. On refroidit le mélange et on ajoute 250 ml d'eau. On décante la phase organique et on la lave à l'eau (3 fois). Un solide précipite de la phase organique. On le recueille par filtration et on le sèche sous pression réduite en présence de pentoxyde de phosphore. Les phases aqueuses sont réunies et agités pendant 7 h environ. Un solide précipite de la phase aqueuse. On le recueille par filtration et on le sèche également sous pression réduite en présence de pentoxyde de phosphore. On réunit les deux précipités et on obtient 16 g de 2,6-dichloro-1*H*-indole-3-carbaldéhyde sous forme d'un solide blanc beige.

H¹ RMN (DMSOd₆, 200MHz) : 9,9 (s, 1H) ; 7,9 (d, 1H) ; 7,4 (s, 1H) ; 7,2 (d, 1H).

### 13.2. 2,6-dichloro-1-méthyl-1H-indole-3-carbaldéhyde

On agite pendant 2 h à reflux une solution de 11 g (52 mmol) de 2,6-dichloro-1*H-*indole-3-carbaldéhyde, obtenu à l'étape 13.1, de 14,7 ml (155 mmol) de sulfate de diméthyle, de 0,62 g (1,8 mmol) d'hydrogénosulfate de tétraméthylammonium, de 770 ml de dichlorométhane et de 27,4 g (258 mmol) de carbonate de potassium dans 120 ml d'eau. On refroidit à température ambiante. On décante la phase organique. On extrait la phase aqueuse avec du dichlorométhane. On réunit les phases organiques et on les lave avec de l'eau puis avec une solution aqueuse saturée en chlorure de sodium. On les sèche sur sulfate de sodium, on les filtre et on les concentre sous pression réduite. On triture le résidu dans de l'éther diisopropylique. On le recueille par filtration et on le reprend dans du dichlorométhane avec un peu de méthanol. On sépare l'insoluble par filtration. On concentre partiellement le filtrat. Un solide précipite, on le recueille par filtration. On réunit l'insoluble et le solide.

On obtient 8,5 g de 2,6-dichloro-1-méthyl-1*H*-indole-3-carbaldéhyde sous forme d'un solide beige pâle.

P.F. : 166-168°C

### 13.3. acide 2,6-dichloro-1-méthyl-1H-indole-3-carboxylique

On introduit 10,5 g (46 mmol) de 2,6-dichloro-1-méthyl-1*H*-indole-3-carbaldéhyde, obtenu selon l'étape 13.2. de l'exemple 13, dans 180 ml de dioxane et 45 ml de 2-méthyl-2-butène. On ajoute lentement une solution de 20 g (221 mmol) de chlorite de sodium et de 20 g (145 mmol) de phosphate monosodique dans 120 ml d'eau. On agite 2 h, puis on ajoute une solution de 5 g (55 mmol) de chlorite de sodium et de 5 g (36 mmol) de phosphate monosodique dans 30 ml d'eau. On agite 2 h, puis on ajoute une solution de 3 g (33 mmol) de chlorite de sodium et de 3 g (22 mmol) de phosphate monosodique dans 20 ml d'eau. On agite 3 h. On ajoute 200 ml d'acétate d'éthyle et on laisse agiter la nuit. On recueille par filtration un insoluble que l'on lave à l'eau. La phase aqueuse est extraite avec de l'acétate d'éthyle. Les phases organiques sont réunies et extraites avec de la soude 1 M. La phase aqueuse est refroidie puis acidifiée par de l'acide chlorhydrique 6 N jusqu'à un pH de 2-3. Un solide précipite. On le recueille par filtration et on le lave à l'eau. On le réunit avec l'insoluble et on les sèche sous pression réduite en présence de pentoxyde de phosphore.

On obtient 8,7 g d'acide 2,6-dichloro-1-méthyl-1*H*-indole-3-carboxylique sous forme de solide blanc jaunâtre.

P.F. : 243 - 245°C

### 13.4. 7-chloro-9-méthyl-2-phényl-3,9-dihydro-4H-pyrimido[4,5-b]indol-4-one

On introduit 12 g (49 mmol) d'acide 2,6-dichloro-1-méthyl-1*H*-indole-3-carboxylique, obtenu selon l'étape 13.3. de l'exemple 13, dans 170 ml de dioxane. On ajoute lentement 58 ml (790 mmol) de chlorure de thionyle et on chauffe le mélange à 70°C pendant 5 h. On refroidit à température ambiante et on concentre le mélange sous pression réduite. On ajoute du toluène puis on concentre sous pression réduite (2 fois). On ajoute ensuite 300 ml de dioxane, on refroidit à 0°C et on ajoute rapidement une solution de 25 g (208 mmol) de benzamidine dans 200 ml de dioxane. On agite le mélange pendant 12 h à température ambiante. On ajoute 50 ml d'eau et 300 ml de dichlorométhane. On décante la phase organique, on la sèche sur sulfate de sodium, on la filtre et on la concentre sous pression réduite. On obtient 30 g d'une huile orangée. On en prend 15 g que l'on ajoute à 300 ml d'éther de diphényle. On chauffe à 200°C pendant 4 h. On refroidit, on recueille par filtration l'insoluble et on le rince avec de l'éther diéthylique. On le verse dans 400 ml de dioxane et on chauffe à reflux. On recueille par filtration à chaud un solide et on le rince avec du dioxane. On le sèche sous pression réduite en présence de pentoxyde de phosphore.

On obtient 6,5 g de 7-chloro-9-méthyl-2-phényl-3,9-dihydro-4*H*-pyrimido[4,5-b]indol-4-one.

P.F. : > 300°C

### 13.5. 4-bromo-7-chloro-9-méthyl-2-phényl-9H-pyrimido[4,5-b]indole

On chauffe à reflux pendant 18 h une solution de 1,5 g (4,8 mmol) de 7-chloro-9-méthyl-2-phényl-3,9-dihydro-4*H*-pyrimido[4,5-b]indol-4-one, obtenu à l'étape 13.4. dans 150 ml d'acétonitrile en présence de 4,0 g (13,9 mmol) d'oxybromure de phosphore et 2,0 g (14,5 mmol) de carbonate de potassium. On refroidit à température ambiante et on verse la solution sur de la glace pilée. On ajoute du dichlorométhane et une solution aqueuse saturée en carbonate de potassium. On filtre la phase organique sur célite™, on la lave à l'eau, on la sèche sur sulfate de sodium, on la filtre et on la concentre sous pression réduite. On purifie le résidu par chromatographie sur colonne de gel de silice avec un mélange de solvants (cyclohexane/dichlorométhane : 100/0 à 50/50). On concentre les fractions contenant le composé sous pression réduite.

On isole 1,1 g de 4-bromo-7-chloro-9-méthyl-2-phényl-9*H*-pyrimido[4,5-b]indole sous forme d'un solide blanc-rosé.

H¹ RMN (CDCl₃, 200MHz) : 8,5-8,4 (m, 2H) ; 8,3 (d, 1H) ; 7,5-7,2 (m, 5H) ; 3,9 (s, 3H).

### 13.6. 2-(7-chloro-9-méthyl-2-phényl-9H-pyrimido[4,5-b]indol-4-yl)-3-(diméthylamino)-3-oxopropanoate d'éthyle

On lave au pentane 0,4 g (10 mmol) d'hydrure de sodium (à 60 % dans de l'huile minérale), puis on ajoute sous azote 50 ml de 1,4-dioxane. On refroidit à 0°C et on ajoute goutte à goutte 1,5 g (9,4 mmol) de 3-(diméthylamino)-3-oxopropanoate d'éthyle. On agite 30 min à température ambiante et on ajoute successivement 0,80 g (2,15 mmol) de 4-bromo-7-chloro-9-méthyl-2-phényl-9*H*-pyrimido[4,5-b]indole, obtenu à l'étape 13.5. et 0,80 g (4,2 mmol) d'iodure cuivreux. On porte la solution à reflux pendant 20 h. On refroidit à température ambiante et on ajoute du dichlorométhane et de l'eau. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 100/0 à 95/5). On concentre les fractions contenant le composé sous pression réduite. On solubilise le résidu dans du dichlorométhane avec de l'acétate d'éthyle. On concentre partiellement le mélange jusqu'à l'apparition d'un précipité. On le recueille par filtration et on le rince avec de l'éther diéthylique.

On isole 1,03 g de 2-(7-chloro-9-méthyl-2-phényl-9*H*-pyrimido[4,5-b]indol-4-yl)-3-(diméthylamino)-3-oxopropanoate d'éthyle contenant minoritairement du 2-(7-chloro-9-méthyl-2-phényl-9*H*-pyrimido[4,5-b]indol-4-yl)-*N*,*N*-diméthylacétamide sous forme d'un solide.

### 13.7. 2-(7-chloro-9-méthyl-2-phényl-9H-pyrimido[4,5-b]indol-4-yl)-N,N-diméthyl-acétamide

On chauffe à 50°C pendant 2 h une solution de 1,03 g (2,3 mmol) de 2-(7-chloro-9-méthyl-2-phényl-9*H*-pyrimido[4,5-b]indol-4-yl)-3-(diméthylamino)-3-oxopropanoate d'éthyle, obtenu à l'étape 13.6, 0,80 g (19 mmol) d'hydroxyde de lithium dans un mélange de 30 ml de tétrahydrofurane, 30 ml de méthanol et 5 ml d'eau. On refroidit le mélange à température ambiante, puis on le concentre sous pression réduite. On ajoute du dichlorométhane, de l'eau et une solution aqueuse 1M d'acide chlorhydrique. On décante la phase organique, on la lave à l'eau, on la sèche sur sulfate de sodium, on la filtre et on la concentre sous pression réduite. On purifie le résidu par chromatographie sur colonne de gel de silice avec un mélange de solvants (dichlorométhane/acétate d'éthyle : 100/0 à 95/5). On concentre les fractions contenant le composé sous pression réduite. On recristallise le résidu dans un mélange dichlorométhane/acétate d'éthyle, on le recueille par filtration, on le rince avec de l'éther éthylique et on le sèche sous pression réduite en présence de pentoxyde de phosphore.

On isole 0,48 g de 2-(7-chloro-9-méthyl-2-phényl-9*H*-pyrimido[4,5-b]indol-4-yl)-*N,N*-diméthylacétamide sous forme d'un solide blanc.

P.F. : 185 - 187°C

LC-MS : M+H = 379

H¹ RMN (CDCl₃, 200MHz) : 8,6 (m, 2H) ; 8,3 (d, 1H) ; 7,5 (m, 4H) ; 7,4 (m, 1H) ; 4,4 (s, 2H) ; 4,0 (s, 3H) ; 3,3 (s, 3H) ; 3,0 (s, 3H).

### Exemple 14 (Composé N°25)

### 7-chloro-9-méthyl-2-phényl-9H-pyrimido[4,5-b]indole-4-carboxylate de méthyle

### 14.1. trifluorométhanesulfonate de 7-chloro-9-méthyl-2-phényl-9H-pyrimido[4,5-b]indol-4-yle

On ajoute goutte à goutte sous azote et à 0°C 2,5 ml (14,56 mmol) d'anhydride trifluorométhanesulfonique à une solution de 3,0 g (9,7 mmol) de 7-chloro-9-méthyl-2-phényl-3,9-dihydro-4*H*-pyrimido[4,5-b]indol-4-one, obtenue à l'étape 13.4. de l'exemple 13, et de 4,3 ml (38,8 mmol) de pyridine dans 100 ml de dichlorométhane. On agite 15 min à 0°C puis 4 h à température ambiante. On ajoute du dichlorométhane, de l'eau et une solution d'acide chlorhydrique 0,1N. On décante la phase organique, on extrait la phase aqueuse avec du dichlorométhane. On réunit les phases organiques. On les sèche sur sulfate de sodium, on les filtre et on les concentre sous pression réduite.

On obtient 3,9 g de trifluorométhanesulfonate de 7-chloro-9-méthyl-2-phényl-9*H-*pyrimido[4,5-b]indol-4-yle sous forme d'un solide blanc-jaunâtre que l'on engage tel que dans l'étape suivante.

### 14.2. 7-chloro-9-méthyl-2-phényl-9H-pyrimido[4,5-b]indole-4-carboxylate de méthyle

On chauffe à 70°C sous atmosphère de monoxyde de carbone pendant 6 h une solution de 2,5 g (5,7 mmol) de trifluorométhanesulfonate de 7-chloro-9-méthyl-2-phényl-9*H*-pyrimido[4,5-b]indol-4-yle, obtenu à l'étape 14.1. de l'exemple 14, 2,2 ml (14,8 mmol) de triéthylamine, 30 mg (0,13 mmol) d'acétate de palladium, 90 mg (0,22 mmol) de 1,3-bis(diphénylphosphino)propane dans 45 ml de *N*,*N-*diméthylformamide et 19 ml de méthanol. On refroidit la solution, on filtre et on rince l'insoluble avec du dichlorométhane. On concentre le filtrat sous pression réduite, puis on le co-évapore avec du toluène sous pression réduite. On ajoute au résidu du dichlorométhane et de l'eau. On sèche la phase organique sur sulfate de sodium, on la filtre et on la concentre sous pression réduite. On purifie le résidu par chromatographie sur colonne de gel de silice avec un mélange de solvants (cyclohexane/dichlorométhane : 50/50 à 0/100). On concentre sous pression réduite les fractions contenant le composé.

On isole 0,58 g de 7-chloro-9-méthyl-2-phényl-9*H*-pyrimido[4,5-b]indole-4-carboxylate de méthyle sous forme d'un solide blanc.

P.F. : 192 - 193°C

H¹ RMN (CDCl₃, 200MHz) : 8,7 (d, 1H) ; 8,6 (m, 2H) ; 7,5-7,4 (m, 4H) ; 7,3 (dd, 1H) ; 4,1 (s, 3H) ; 3,9 (s, 3H).

### Exemple 15 (Composé N°26)

### Acide 7-chloro-9-méthyl-2-phényl-9H-pyrimido[4,5-b]indole-4-carboxylique

On agite 12 h à température ambiante une solution de 0,57 g (1,62 mmol) de 7-chloro-9-méthyl-2-phényl-9*H*-pyrimido[4,5-b]indole-4-carboxylate de méthyle, obtenu à l'étape 14.2 de l'exemple 14, et de 300 mg (7,1 mmol) d'hydroxyde de lithium monohydrate dans un mélange de 50 ml de tétrahydrofurane, 20 ml de méthanol et 10 ml d'eau. On ajoute une solution 1 N d'acide chlorhydrique jusqu'à un pH d'environ 1. On élimine sous pression réduite les solvants organiques et on recueille par filtration le précipité obtenu. On le rince avec de l'eau puis de l'éther diéthylique et on le sèche sous pression réduite en présence de pentoxyde de phosphore.

On isole 0,5 g d'acide 7-chloro-9-méthyl-2-phényl-9*H*-pyrimido[4,5-b]indole-4-carboxylique sous forme d'un solide blanc.

P.F. : 208 - 209 °C

H¹ RMN (DMSOd₆, 200MHz) : 8,6-8,4 (m, 3H) ; 7,8 (d, 1H) ; 7,5-7,4 (m, 3H) ; 7,3 (dd, 1H) ; 3,9 (s, 3H).

### Exemple 16 (Composé N°2)

### 7-chloro-N,N,9-triméthyl-2-phényl-9H-pyrimido[4,5-b]indole-4-carboxamide

On chauffe à 70°C sous atmosphère d'azote pendant 3 h une solution de 0,60 g (1,8 mmol) d'acide 7-chloro-9-méthyl-2-phényl-9*H*-pyrimido[4,5-b]indole-4-carboxylique, obtenu selon l'exemple 15, et 0,45 g (2,7 mmol) de carbonyldiimidazole dans 100 ml de tétrahydrofurane. On refroidit à température ambiante et on sature le milieu réactionnel avec de la diméthylamine gazeuse jusqu'à l'obtention d'un pH basique, puis on agite pendant 12 h à température ambiante. On concentre la solution sous pression réduite. On ajoute ensuite du dichlorométhane et une solution 0,5 N d'acide chlorhydrique. On lave la phase organique avec une solution aqueuse saturée en chlorure de sodium, on la sèche sur sulfate de sodium, on la filtre et on la concentre sous pression réduite. On purifie le résidu par chromatographie sur colonne de gel de silice avec un mélange de solvants (dichlorométhane/méthanol : 100/0 à 95/5). On concentre les fractions contenant le composé sous pression réduite. On recristallise le solide dans de l'acétate d'éthyle et on le rince avec de l'éther diéthylique.

On isole 0,33 g de 7-chloro-*N*,*N*,9-triméthyl-2-phényl-9*H*-pyrimido(4,5-b]indole-4-carboxamide sous forme d'un solide blanc.

P.F. : 211 - 213°C

LC-MS : M+H = 365

H¹ RMN (CDCl₃, 200MHz) : 8,7 (m, 2H) ; 8,1 (d, 1H) ; 8,6-8,5 (m, 4H) ; 7,3 (dd, 1H) ; 4,0 (s, 3H) ; 3,4 (s, 3H) ; 3,1 (s, 3H).

### Exemple 17 (Composé N°28)

### 7-chloro-2-(6-méthoxypyridin-3-yl)-N,N,9-triméthyl-9H-pyrimido[4,5-b]indole-4-carboxamide

On chauffe à 130°C pendant 2 h une solution de 490 mg (3,24 mmol) de 6-méthoxypyridine-3-carboxamidine et 300 mg (1,0 mmol) de 2-(2,6-dichloro-1-méthyl-1*H*-indol-3-yl)-*N*,*N*-diméthyl-2-oxoacétamide, obtenu à l'étape 1.2. de l'exemple 1, dans 7 ml de *N,N*-diméthylformamide. On refroidit la solution. On recueille le précipité par filtration, on le rince avec du 1,4-dioxanne et on le sèche sous pression réduite. On le purifie par chromatographie sur colonne de gel de silice avec un mélange de solvants (dichlorométhane / méthanol : 97/3). On recristallise le composé obtenu dans un mélange acétate d'éthyle / méthanol, on le filtre, on le rince avec de l'éther diéthylique et on le sèche sous pression réduite.

On isole 0,22 g de 7-chloro-2-(6-méthoxypyridin-3-yl)-*N,N*,9-triméthyl-9*H-*pyrimido[4,5-b]indole-4-carboxamide sous forme d'un solide blanc.

P.F. : 267-268°C

LC-MS : M+H = 396

H¹ RMN (CDCl₃, 200MHz) : 9,45 (d, 1H) ; 8,75 (dd, 1H) ; 8,05 (d, 1H) ; 7,5 (d, 1H) ; 7,25 (dd, 1H) ; 6,85 (d,1H) ; 4,05 (s, 3H) ; 4,0 (s, 3H) ; 3,35 (s, 3H) ; 3,1 (s, 3H).

### Exemple 18 (Composé N°41)

### 7-chloro-2-(5-éthylpyridin-3-yl)-N,N,9-triméthyl-9H-pyrimido[4,5-b]indole-4-carboxamide

On chauffe à 130°C pendant 3 h une solution de 820 mg (5,50 mmol) de 5-éthylpyridin-3-carboxamidine et 400 mg (1,34 mmol) de 2-(2,6-dichloro-1-méthyl-1*H*-indol-3-yl)-*N,N*-diméthyl-2-oxoacétamide, obtenu à l'étape 1.2 de l'exemple1, dans 30 ml de *N,N*-diméthylformamide. On refroidit la solution. On concentre le mélange réactionnel sous pression réduite. On ajoute du dichlorométhane et on lave à l'eau. On sèche la phase organique sur sulfate de sodium. On filtre et on concentre sous pression réduite. On purifie le résidu par chromatographie sur colonne de gel de silice avec un mélange de solvants (dichlorométhane / acétate d'éthyle : 90/10 à 80/20, puis acétate d'éthyle / méthanol : 100/0 à 95/5). On recristallise le composé obtenu dans un mélange dichlorométhane / acétate d'éthyle, on le filtre, on le rince avec de l'acétate d'éthyle et on le sèche sous pression réduite.

On isole 0,18 g de 7-chloro-2-(5-éthylpyridin-3-yl)-*N,N*,9-triméthyl-9*H-*pyrimido[4,5-*b*]indole-4-carboxamide sous forme d'un solide jaune pâle.

P.F. : 218 -219°C

LC-MS : M+H = 394

H¹ RMN (CDCl₃, 200MHz) : 9,45 (d, 1H) ; 8,60 (m, 2H) ; 7,95 (d, 1H) ; 7,90 (d, 1H) : 7,4 (dd, 1H) ; 4,0 (s, 3H) ; 3,30 (s, 3H) ; 3,20 (s, 3H) ; 2,95 (s, 3H) ; 2.80 (q, 2H) ; 1,25 (t, 3H).

### Exemple 19 (Composé N°48)

### chlorure de 5-(7-chloro-4-diméthylcarbamoyl-9-méthyl-9H-pyrimido[4,5-b]indol-2-yl)-2,3-diméthyl-pyridinium

On chauffe à reflux pendant 5 h une solution de 930 mg (6,23 mmol) de 5,6-diméthylpyridin-3-carboxamidine et 600 mg (2,01 mmol) de 2-(2,6-dichloro-1-méthyl-1*H*-indol-3-yl)-*N,N*-diméthyl-2-oxoacétamide, obtenu à l'étape 1.2. de l'exemple 1, dans 50 ml de o-xylène. On refroidit la solution. On concentre le mélange réactionnel sous pression réduite. On ajoute du dichlorométhane et une solution aqueuse saturée en bicarbonate de sodium. On lave la phase organique avec l'eau, on la sèche sur sulfate de sodium. On la filtre et on concentre le filtrat sous pression réduite. On purifie le résidu par chromatographie sur colonne de gel de silice avec un mélange de solvants (dichlorométhane / acétate d'éthyle : de 80/20 à 0/100, puis acétate d'éthyle / méthanol : 100/0 à 95/5). Après évaporation des solvants, on récupère 340 mg (0,86 mmol) d'un solide blanc. On le dissout dans du dichlorométhane et on ajoute 0,35 ml (1,73 mmol) d'une solution 5-6 N d'acide chlorhydrique dans de l'isopropanol. On évapore les solvants sous pression réduite et on recristallise le résidu dans de l'éthanol absolu. On le filtre, on le rince avec de l'éther diéthylique et on le sèche sous pression réduite.

On isole 0,30 g de chlorure de 5-(7-chloro-4-diméthylcarbamoyl-9-méthyl-9*H-*pyrimido[4,5-b]indol-2-yl)-2,3-diméthyl-pyridinium.

P.F. : 285 - 289°C

LC-MS : M+H = 394

H¹ RMN (CDCl₃, 200MHz) : 9,4 (s, 1H) ; 9,05 (s, 1H) ; 8,0 (d, 1H) ; 7,90 (d, 1H) ; 7,45 (dd, 1H) ; 4,05 (s, 3H) ; 3,20 (s, 3H) ; 2,95 (s, 3H) ; 2,70 (s, 3H) ; 2,50 (s, 3H).

### Exemple 20 (Composé N°49)

### Chlorure de 3-(7-chloro-4-diméthylcarbamoyl-9-méthyl-9H-pyrimido[4,5-b]indol-2-yl)-5,6,7,8-tétrahydro-quinolinium

On chauffe à reflux pendant 5 h une solution de 2,40 g (13,7 mmol) de 5,6,7,8-tétrahydroquinolin-3-carboxamidine et 800 mg (2,67 mmol) de 2-(2,6-dichloro-1-méthyl-1*H*-indol-3-yl)-*N,N*-diméthyl-2-oxoacétamide, obtenu à l'étape 1.2 de l'exemple 1, dans 70 ml de o-xylène. On refroidit la solution. On concentre le mélange réactionnel sous pression réduite. On ajoute du dichlorométhane et une solution aqueuse saturée en bicarbonate de sodium. On lave la phase organique à l'eau, on la sèche sur sulfate de sodium. On la filtre et on concentre le filtrat sous pression réduite. On purifie le résidu par chromatographie sur colonne de gel de silice avec un mélange de solvants (dichlorométhane / acétate d'éthyle : de 90/10 à 0/100). Après évaporation des solvants, on récupère 870 mg (2,07 mmol) d'un solide blanc. On le dissout dans du dichlorométhane et on ajoute 0,83 ml (4,14 mmol) d'une solution 5-6 N d'acide chlorhydrique dans de l'isopropanol. On évapore les solvants sous pression réduite et on recristallise le résidu dans de l'éthanol absolu. On le filtre, on le rince avec de l'éther diéthylique et on le sèche sous pression réduite.

On isole 0,75 g de chlorure de chlorure de 3-(7-chloro-4-diméthylcarbamoyl-9-méthyl-9*H*-pyrimido[4,5-b]indol-2-yl)-5,6,7,8-tétrahydro-quinolinium.

P.F. : 289 - 291°C

LC-MS : M+H = 420

H¹ RMN (CDCl₃, 200MHz) : 9,35 (s, 1H) ; 8,60 (s, 1H) ; 7,90 (d, 1H) ; 7,85 (d, 1H) ; 7,35 (dd, 1H) ; 4,00 (s, 3H) ; 3,20 (s, 3H) ; 2,95-2,80 (m, 7H) ; 1,95-1,65 (m, 4H).

### Exemple 21 (Composé N°50)

### Chlorure de 4-(7-chloro-4-diméthylcarbamoyl-9-méthyl-9H-pyrimido[4,5-b]indol-2-yl)-2,6-diméthyl-pyridinium

On chauffe à reflux pendant 5 h une solution de 0,78 g (5,23 mmol) de 2,6-diméthylpyridin-4-carboxamidine et 700 mg (2,34 mmol) de 2-(2,6-dichloro-1-méthyl-1*H*-indol-3-yl)-*N*,*N*-diméthyl-2-oxoacétamide, obtenu à l'étape 1.2 de l'exemple 1, dans 30 ml de o-xylène. On refroidit la solution. On concentre le mélange réactionnel sous pression réduite. On ajoute du dichlorométhane, de l'eau et une solution aqueuse 1M d'hydroxyde de sodium. On lave la phase organique avec l'eau, on la sèche sur sulfate de sodium. On la filtre et on concentre le filtrat sous pression réduite. On purifie le résidu par chromatographie sur colonne de gel de silice avec un mélange de solvants (dichlorométhane / acétate d'éthyle : de 80/20 à 0/100, puis acétate d'éthyle / méthanol : 100/0 à 95/5). Après évaporation des solvants, on récupère 540 mg (1,37 mmol) d'un solide blanc. On le dissout dans du dichlorométhane et on ajoute 0,55 ml (2,74 mmol) d'une solution 5-6 N d'acide chlorhydrique dans de l'isopropanol. On évapore les solvants sous pression réduite et on recristallise le résidu dans de l'éthanol absolu. On le filtre, on le rince avec de l'éther diéthylique et on le sèche sous pression réduite.

On isole 0,69 g de chlorure de chlorure de 4-(7-chloro-4-diméthylcarbamoyl-9-méthyl-9*H*-pyrimido[4,5-*b*]indol-2-yl)-2,6-diméthyl-pyridinium.

P.F. : 288 - 292°C

LC-MS : M+H = 394

H¹ RMN (CDCl₃, 200MHz) : 8,50 (s, 2H) ; 8,00 (d, 1H) ; 7,95 (d, 1H) ; 7,45 (dd, 1H) ; 4,05 (s, 3H) ; 3,20 (s, 3H) ; 2,95 (s, 3H) ; 2,75 (s, 6H).

Le tableau 1 qui suit illustre les structures chimiques et les propriétés physiques de quelques composés de l'invention.

Dans la colonne « sel » de ce tableau, « - » représente un composé sous forme de base libre, alors que « HCl » représente un composé sous forme de chlorhydrate et le rapport entre parenthèses est le rapport (acide:base).

**Tableau 1**

| 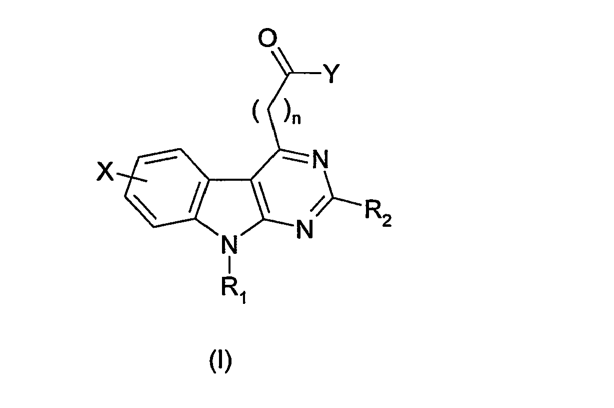 | | | | | | | |
|---|---|---|---|---|---|---|---|
| **N°** | **n** | **X** | **Y** | **R₁** | **R₂** | **Sel** | **P.F. (°C)** |
| 1 | 0 | H | N(CH₃)₂ | CH₃ | phényl | - | 193 - 195 |
| 2 | 0 | 7-Cl | N(CH₃)₂ | CH₃ | phényl | - | 211 - 213 |
| 3 | 0 | 7-F | N(CH₃)₂ | CH₃ | phényl | - | 229 - 231 |
| 4 | 0 | 6-Cl | N(CH₃)₂ | CH₃ | phényl | - | 222 - 224 |
| 5 | 0 | 7-Cl | N(CH₃)₂ | H | phényl | - | 232 - 233 |
| 6 | 0 | 7-Cl | N(CH₂CH₃)₂ | CH₃ | phényl | - | 174 - 176 |
| 7 | 0 | 7-Cl | pyrrolidin-1-yl | CH₃ | phényl | - | 210 - 212 |
| 8 | 0 | 7-Cl | pipéridin-1-yl | CH₃ | phényl | - | 216 - 218 |
| 9 | 0 | 7-Cl | morpholin-1-yl | CH₃ | phényl | - | 214 - 216 |
| 10 | 0 | 7-Cl | 4-(*N*-azétidinyl) pipéridin-1-yl | CH₃ | phényl | - | 248 - 249 |
| 11 | 0 | 7-Cl | *N*-méthylpipérazin-1-yl | CH₃ | phényl | - | 236 - 238 |
| 12 | 0 | 6-Cl | N(CH₃)₂ | CH₃ | pyridin-4-yl | - | 226 - 227 |
| 13 | 0 | 7-F | N(CH₃)₂ | CH₃ | pyridin-4-yl | - | 272 - 274 |
| 14 | 0 | 7-Cl | pyrrolidin-1-yl | CH₃ | pyridin-4-yl | - | 256 - 258 |
| 15 | 0 | 7-Cl | N(CH₃)₂ | CH₃ | pyridin-4-yl | - | 268 - 269 |
| 16 | 0 | 7-Cl | N(CH₃)₂ | CH₃ | pyridin-3-yl | - | 283 - 284 |
| 17 | 0 | 7-Cl | N(CH₃)₂ | CH₃ | pyridin-2-yl | - | 233 - 235 |
| 18 | 0 | 7-Cl | N(CH₃)₂ | CH₃ | pyrazin-2-yl | - | 272 - 273 |
| 19 | 1 | 7-Cl | N(CH₃)₂ | CH₃ | phényl | - | 185 - 187 |
| 20 | 1 | 6-Cl | N(CH₃)₂ | CH₃ | phényl | - | 234 - 236 |
| 21 | 1 | 7-Cl | N(CH₃)₂ | CH₃ | pyridin-4-yl | - | 231 - 232 |
| 22 | 1 | 7-Cl | N(CH₃)₂ | CH₃ | pyridin-3-yl | - | 248 - 251 |
| 23 | 0 | H | N(CH₃)₂ | H | phényl | - | 218 - 219 |
| 24 | 0 | 7-Cl | O(CH₂CH₃) | CH₃ | phényl | - | 139 - 140 |
| 25 | 0 | 7-Cl | OCH₃ | CH₃ | phényl | - | 192 - 193 |
| 26 | 0 | 7-Cl | OH | CH₃ | phényl | - | 208 - 209 |
| 27 | 0 | 7-Cl | N(CH₃)₂ | CH₃ | 6-méthyl-pyridin-3-yl | - | 276 - 277 |
| 28 | 0 | 7-Cl | N(CH₃)₂ | CH₃ | 6-méthoxy-pyridin-3-yl | - | 267 - 268 |
| 29 | 0 | 7-Cl | N(CH₃)₂ | CH₃ | 2-méthyl-pyridin-4-yl | - | 242 - 244 |
| 30 | 0 | 7-Cl | N(CH₃)₂ | CH₃ | 2-méthoxy-pyridin-4-yl | - | 278 - 279 |
| 31 | 0 | 7-Cl | N(CH₃)₂ | Isobutyl | pyridin-4-yl | HCl (1:1) | 230 - 236 |
| 32 | 0 | 7-Cl | N(CH₃)₂ | CH₃ | cyclopropyl | - | 197 - 198 |
| 33 | 0 | 7-Cl | N(CH₃)₂ | CH₃ | méthyl | - | 193 -195 |
| 34 | 0 | 7-Cl | N(CH₃)₂ | CH₃ | isopropyl | - | 125 - 126 |
| 35 | 0 | 7-Cl | N(CH₃)₂ | CH₃ | tétrahydro-2*H*-pyran-4-yl | - | 174.5-175.5 |
| 36 | 0 | 7-Cl | N(CH₃)₂ | CH₃ | 4-[(diméthylamino)méthyl]phényl | HCl (1:1) | 257 - 258 |
| 37 | 0 | 7-Cl | N(CH₃)₂ | CH₃ | 6-chloropyridin-3-yl | - | 275 - 276 |
| 38 | 0 | 7-Cl | N(CH₃)₂ | CH₃ | 6-(trifluorométhyl)pyridin-3-yl | - | 253 - 255 |
| 39 | 0 | 7-Cl | N(CH₃)₂ | CH₃ | 6-éthoxypyridin-3-yl | - | 237 - 239 |
| 40 | 0 | 7-Cl | N(CH₃)₂ | CH₃ | 6-éthylpyridin-3-yl | - | 232 - 235 |
| 41 | 0 | 7-Cl | N(CH₃)₂ | CH₃ | 5-éthylpyridin-3-yl | - | 218 - 219 |
| 42 | 0 | 7-Cl | N(CH₃)₂ | CH₃ | 5-méthylpyridin-3-yl | - | 313 - 314 |
| 43 | 0 | 7-Cl | N(CH₃)₂ | CH₃ | pyrimidin-5-yl | - | 322 - 325 |
| 44 | 0 | 7-Cl | N(CH₃)₂ | CH₃ | 5-méthoxypyridin-3-yl | - | 258 - 260 |
| 45 | 0 | 7-Cl | N(CH₃)₂ | CH₃ | 6-(méthoxyméthyl)pyridin-3-yl | - | 239 - 241 |
| 46 | 0 | 7-Cl | N(CH₃)₂ | CH₃ | 2-méthyl-1,3-thiazol-4-yl | - | 256 - 258 |
| 47 | 0 | 7-Cl | N(CH₃)₂ | CH₃ | 6-[(diméthylamino)méthyl]-pyridin-3-yl | HCl(1:1) | 240 - 242 |
| 48 | 0 | 7-Cl | N(CH₃)₂ | CH₃ | 5,6-diméthylpyridin-3-yl | HCl(1:1) | 285 - 289 |
| 49 | 0 | 7-Cl | N(CH₃)₂ | CH₃ | 5,6,7,8-tétrahydroquinolin-3-yl | HCl (1:1) | 289 - 291 |
| 50 | o | 7-Cl | N(CH₃)₂ | CH₃ | 2,6-diméthylpyridin-4-yl | HCl (1:1) | 288 - 292 |

Les composés de l'invention ont été soumis à des essais pharmacologiques qui ont mis en évidence leur intérêt comme substances à activités thérapeutiques.

### Etude de la liaison [³H]Ro5-4864 aux récepteurs de type périphérique aux benzodiazépines (sites p ou PBR)

L'affinité des composés de l'invention pour les sites p ou PBR (sites de liaison de type périphérique aux benzodiazépines) a été déterminée.

Les récepteurs sites p peuvent être marqués sélectivement dans des membranes de rein de rat incubées en présence de [³H]Ro5-4864. Les composés de l'invention ont fait l'objet d'une étude *in vitro* quant à leur affinité pour ces récepteurs.

Les animaux utilisés sont des rats mâles Sprague Dawley (Iffa Credo) de 180 à 300 mg. Après décapitation, le rein est prélevé et le tissu est homogénéisé à 4°C au moyen d'un homogénéiseur Polytron™ pendant 2 min à 6/10 de la vitesse maximale, dans 35 volumes de tampon phosphate Na₂HPO₄ 50 mM, à un pH ajusté à 7,5 avec du NaH₂PO₄. L'homogénat membranaire est filtré sur gaze et dilué 10 fois avec du tampon.

Le [³H]Ro5-4864 (Activité spécifique : 70-90 Ci/mmol ; New England Nuclear), à une concentration de 0,5 nM, est incubé en présence de 100 µl de l'homogénat membranaire dans un volume final de 1 ml de tampon contenant le composé à tester.

Après une incubation de 3 h à 0°C, les membranes sont récupérées par filtration sur filtres Whatman GF/B™ lavés avec 2 fois 4,5 ml de tampon d'incubation froid (0°C). La quantité de radioactivité retenue par le filtre est mesurée par scintigraphie liquide.

Pour chaque concentration de composé étudié, le pourcentage d'inhibition de la liaison du [³H]Ro5-4864, puis la concentration Cl₅₀, concentration qui inhibe 50 % de la liaison spécifique, sont déterminés.

Les Cl₅₀ des composés les plus actifs de l'invention présentent des valeurs allant de 1 nM à 200 nM.

Le tableau 2 ci-dessous présente les Cl₅₀ de quelques composés selon l'invention.

**Tableau 2**

| Composé N° | Cl₅₀ |
|---|---|
| 2 | 2,2 nM |
| 15 | 3,1 nM |
| 19 | 1,3 nM |

Les composés de l'invention sont donc des ligands affins pour les récepteurs de type périphérique aux benzodiazépines.

### Etude de l'activité neurotrophe

### Test de survie des motoneurones après section du nerf facial chez le rat âgé de 4 jours

Après lésion du nerf facial chez le rat immature, les motoneurones du noyau facial subissent une mort neuronale par apoptose. L'évaluation de la survie neuronale est réalisée à l'aide de méthodes histologiques et comptage neuronal. Des rats immatures de 4 jours sont anesthésiés au pentobarbital (3 mg/kg par voie i.p.). Le nerf facial droit est dégagé et sectionné, à sa sortie du foramen stylomastoïdien. Après le réveil, les ratons sont remis avec leur mère et traités, pendant 7 jours, par une ou deux administrations quotidiennes, par voie orale ou intrapéritonéale, à des doses allant de 1 à 10 mg/kg. 7 jours après la lésion, les animaux sont décapités, et les cerveaux congelés dans l'isopentane à - 40°C. Le noyau facial est coupé au cryostat, en sections de 10 µm, dans sa totalité. Les motoneurones sont colorés au crésyl violet et comptés à l'aide du logiciel Histo™ (Biocom™).

Dans ce modèle, les composés de l'invention les plus actifs augmentent la survie neuronale d'environ 10 à 40 %.

Le tableau 3 ci-dessous présente les résultats pour quelques composés selon l'invention dans ce modèle.

**Tableau 3**

| Composé N° | % |
|---|---|
| 2 | +25* |
| 15 | +40* |
| 19 | +21* |

| | |
|---|---|
| * par voie orale, à 10mg/kg | |

Les résultats des essais montrent que les composés les plus actifs de l'invention favorisent la régénération nerveuse.

Les composés de l'invention peuvent donc être utilisés pour la préparation de médicaments destinés à prévenir ou à traiter une pathologie dans laquelle les récepteurs de type périphérique aux benzodiazépines sont impliqués.

Ces médicaments trouvent leur emploi en thérapeutique, notamment dans le traitement et/ou la prévention des neuropathies périphériques de différents types, comme les neuropathies traumatiques ou ischémiques, neuropathies diabétiques, infectieuses, alcooliques, médicamenteuses ou génétiques, ainsi que des affections du motoneurone, telle que les amyotrophies spinales et la sclérose latérale amyotrophique. Ces médicaments trouveront également une application dans le traitement des maladies neurodégénératives du système nerveux central, soit de type aigu comme les accidents vasculaires cérébraux et les traumatismes crâniens et médullaires, soit de type chronique comme les maladies auto-immunes (sclérose en plaques), la maladie d'Alzheimer, la maladie de Parkinson et toute autre maladie dans laquelle l'administration de facteurs neurotrophes a un effet thérapeutique.

Les composés de l'invention peuvent également être utilisés pour la préparation de médicaments destinés à la prévention et/ou au traitement de l'anxiété, de l'épilepsie et des troubles du sommeil. En effet, les ligands des sites p ou PBR stimulent la production de neurostéroides tels que la pregnénolone, la déhydroepiandrostérone, et la 3-alphahydroxy-5-alphaprégnan-20-one en favorisant le transfert du cholestérol de l'extérieur à l'intérieur de la membrane mitochondriale. Ces neurostéroïdes modulent l'activité du complexe macromoléculaire GABA_{A}-canal chlorure et ainsi peuvent produire des activités anxiolytiques, anti-convulsivantes et sédatives.

Les composés de l'invention peuvent aussi être utilisés dans les traitements de l'insuffisance rénale aiguë ou chronique, de la glomérulonéphrite, de la néphropathie diabétique, de l'ischémie et de l'insuffisance cardiaques, de l'infarctus du myocarde, de l'ischémie des membres inférieurs, du vasospasme coronaire, de l'angine de poitrine, des pathologies associées aux valves cardiaques, des maladies cardiaques inflammatoires, des effets secondaires dus à des médicaments cardiotoxiques ou aux suites d'une chirurgie cardiaque, de l'athérosclérose et de ses complications thrombo-emboliques, de la resténose, des rejets de greffes, des conditions liées à une prolifération ou une migration incorrectes des cellules musculaires lisses.

Par ailleurs, des données récentes de la littérature indiquent que le récepteur de type périphérique aux benzodiazépines pourrait jouer un rôle fondamental dans la régulation de la prolifération cellulaire et les processus de cancérisation. D'une manière générale, et par comparaison avec des tissus normaux, une densité accrue de récepteurs de type périphérique aux benzodiazépines est observée dans différents types de tumeurs et cancers.

Dans les astocytomes humains, le niveau d'expression du récepteur de type périphérique aux benzodiazépines est corrélé avec le degré de malignité de la tumeur, l'index de prolifération et la survie des patients. Dans les tumeurs cérébrales humaines, l'augmentation du nombre de récepteurs de type périphérique aux benzodiazépines est utilisée comme une indication diagnostique en imagerie médicale et comme cible thérapeutique pour des conjugués formés d'un ligand du récepteur de type périphériques aux benzodiazépines et d'une drogue cytostatique. Une densité élevée de récepteurs de type périphérique aux benzodiazépines est également observée dans les carcinomes ovariens et les cancers du sein. Concernant ces derniers, il a été démontré que le niveau d'expression des récepteurs de type périphérique aux benzodiazépines est relié au potentiel agressif de la tumeur; de plus la présence d'un agoniste du récepteur de type périphérique aux benzodiazépines stimule la croissance d'une lignée de cancer mammaire.

L'ensemble de ces résultats, qui suggère une fonction délétère du récepteur de type périphérique aux benzodiazépines dans les processus de cancérisation, constitue une base pertinente pour la recherche de ligands synthétiques spécifiques du récepteur de type périphérique aux benzodiazépines capables d'en bloquer les effets.

Les composés peuvent donc être utilisés pour le traitement des tumeurs et cancers.

Les récepteurs de type périphérique aux benzodiazépines sont également présents au niveau de la peau et, à ce titre, les composés utilisables selon l'invention peuvent être utilisés pour la prophylaxie ou le traitement des stress cutanés.

Par stress cutané, on entend les différentes situations qui pourraient provoquer des dommages en particulier au niveau de l'épiderme, quel que soit l'agent qui provoque ce stress. Cet agent peut être interne et/ou externe à l'organisme, comme un agent chimique ou radicalaire, ou bien externe, comme un rayonnement ultraviolet.

Ainsi les composés utilisables selon l'invention sont destinés à prévenir et à lutter contre les irritations cutanées, les dartres, les érythèmes, les sensations dysesthésiques, les sensations d'échauffement, les prurits de la peau et/ou des muqueuses, le vieillissement et peuvent aussi être utilisés dans les désordres cutanés tels que, par exemple, le psoriasis, les maladies prurigineuses, l'herpès, les photodermatoses, les dermatites atopiques, les dermatites de contact, les lichens, les prurigos, les prurits, les piqûres d'insectes, dans les fibroses et autres troubles de la maturation des collagènes, dans les désordres immunologiques ou encore dans des affections dermatologiques comme l'eczéma.

Les composés de l'invention peuvent également être utilisés pour la prévention et le traitement des maladies inflammatoires chroniques, notamment la polyarthrite rhumatoïde, et des maladies inflammatoires pulmonaires, en particulier l'asthme, le syndrome de détresse respiratoire aiguë (ARDS, acute respiratory distress syndrome) et la maladie obstructive du poumon (COPD, chronic obstructive pulmonary disease), la mucoviscidose, les maladies bronchopulmonaires, les pneumopathies, la fibrose pulmonaire).

Selon un de ses aspects, l'invention a pour objet des médicaments qui comprennent un composé de formule (I), ou un sel d'addition de ce dernier à un acide pharmaceutiquement acceptable, ou encore un hydrate ou un solvate.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

1. Composé répondant à la formule (I) 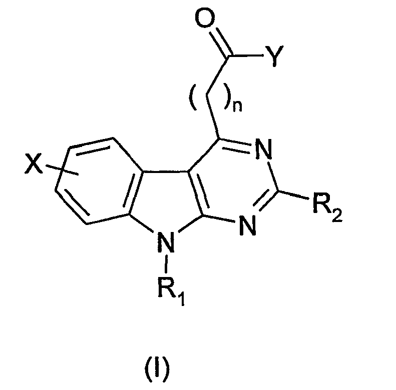 dans laquelle
n représente le chiffre 0 ou 1 ;
X représente un atome d'hydrogène ou d'halogène ;
Y représente un groupe OR₃ ou un groupe NR₄R₅ ;
R₁ représente un atome d'hydrogène ou un groupe (C₁-C₆)alkyle ;
R₂ représente un groupe (C₁-C₆)alkyle, un phényle ou un hétérocycle monocyclique ou bicyclique, lesdits groupes phényle ou hétérocycle pouvant porter un ou plusieurs atomes d'halogène et/ou un ou plusieurs groupes (C₁-C₆)alkyle, (C₁-C₆)alcxyle, (C₁-C₆)alcoxy-(C₁-C₆)alkyle, (C₁-C₆)alkylamino-(C₁-C₆)alkyle ou (C₁-C₆)dialkylamino-(C₁-C₆)alkyle ;
R₃ représente un atome d'hydrogène, un groupe (C₁-C₆)alkyle ou un benzyle ;
R₄ et R₅ représentent chacun, indépendamment l'un de l'autre, un atome d'hydrogène ou un groupe (C₁-C₆)alkyle, ou bien R₄ et R₅ forment, avec l'atome d'azote qui les porte, un groupe aziridinyle, azétidinyle, pyrrolidinyle, pipéridinyle, morpholinyle, thiomorpholinyle, ou pipérazinyle, éventuellement substitué par un (C₁-C₆)alkyle, phényle ou hétérocycle;
à l'état de base ou de sel d'addition à un acide, ainsi qu'à l'état d'hydrate ou de solvate.

2. Composé de formule (I) selon la revendication 1, **caractérisé en ce que** :
n représente le chiffre 0 ou 1 ;
X représente un atome d'hydrogène ou d'halogène ;
Y représente un groupe OR₃ ou un groupe NR₄R₅ ;
R₁ représente un atome d'hydrogène ou un groupe (C₁-C₆)alkyle ;
R₂ représente un phényle ou un hétérocycle de type pyridinyle, pyrimidinyle, pyrazinyle, pyridazinyle, le phényle ou l'hétérocycle pouvant porter un ou plusieurs atomes d'halogène et/ou un ou plusieurs groupes (C₁-C₆)alkyle, (C₁-C₆)alcoxyle ;
R₃ représente un hydrogène, un (C₁-C₆)alkyle ou un benzyle ; et
R₄ et R₅ représentent chacun, indépendamment l'un de l'autre, un atome d'hydrogène ou un groupe (C₁-C₆)alkyle, ou bien R₄ et R₅ forment, avec l'atome d'azote qui les porte, un groupe aziridinyle, azétidinyle, pyrrolidinyle, pipéridinyle, morpholinyle, thiomorpholinyle, ou pipérazinyle, éventuellement substitué par un (C₁-C₆)alkyle, phényle ou azétidinyle ;
à l'état de base ou de sel d'addition à un acide, ainsi qu'à l'état d'hydrate ou de solvate.

3. Composé de formule (I) selon la revendication 1, **caractérisé en ce que** :
n représente le chiffre 0 ou 1;
X représente un atome d'hydrogène ou de fluor ou de chlore ;
Y représente un groupe hydroxy, OCH₃, O(CH₂CH₃), N(CH₃)₂, N(CH₂CH₃)₂, pyrrolidinyle, pipéridinyle, morpholinyle, (*N*-azétidinyl)-pipéridinyle ou *N-*méthylpipérazinyle;
R₁ représente un atome d'hydrogène, un groupe méthyle ou isobutyle ;
R₂ représente un phényle, un groupe méthyle, isopropyle, cyclopropyle ou un hétérocycle de type pyridinyle, pyrazinyle, pyrimidinyle, thiazolyle, tétrahydroquinolinyle, tétrahydropyranyle, les groupes phényle et hétérocycle pouvant éventuellement être substitués par un ou plusieurs groupes halogène et/ou un ou plusieurs groupes (C₁-C₆)alkyle, (C₁-C₆)alcoxyle, (C₁-C₆)alcoxy-(C₁-C₆)alkyle, (C₁-C₆)dialkylamino, (C₁-C₆)dialkylamino-(C₁-C₆)alkyle ;
à l'état de base ou de sel d'addition à un acide, ainsi qu'à l'état d'hydrate ou de solvate.

4. Composé de formule (I) selon la revendication 1 ou 3, choisi parmi :
*N*,*N*,9-triméthyl-2-phényl-9*H*-pyrimido[4,5*-b*]indole-4-carboxamide
7-*chloro*-*N*,*N*,9-triméthyl-2-phényl-9*H*-pyrimido[4,5*-b*]indole-4-carboxamide
7-*fluoro*-*N*,*N*,9-triméthyl-2-phényl-9*H*-pyrimido[4,5*-b*]indole-4-carboxamide
6-*chloro*-*N*,*N*,9-triméthyl-2-phényl-9*H*-pyrimido[4,5*-b*]indole-4-carboxamide
7-chloro-*N*,*N*-diméthyl-2-phényl-9*H*-pyrimido[4,5*-b*]indole-4-carboxamide
7-chloro-*N*,*N*-diéthyl-9-méthyl-2-phényl-9*H*-pyrimido[4,5*-b*]indole-4-carboxamide
7-chloro-9-méthyl-2-phényl-4-(pyrrolidin-1-ylcarbonyl)-9*H*-pyrimido[4,5*-b*]indole
7-chloro-9-méthyl-2-phényl-4-(pipéridin-1-ylcarbonyl)-9*H*-pyrimido[4,5*-b*]indole 7-chloro-9-méthyl-4-(morpholin-4-ylcarbonyl)-2-phényl-9*H*-pyrimido[4,5*-b*]indole 4-[(4-azétidin-1-yl)pipéridin-1-ylcarbonyl]-7-chloro-9-méthyl-2-phényl-9*H*-pyrimido-[4,5*-b*]indole
7-chloro-9-méthyl-4-[(4-méthylpipérazin-1-yl)carbonyl]-2-phényl-9*H*-pyrimido[4,5-b]indole
6-chloro-*N*,*N*,9-triméthyl-2-pyridin-4-yl-9*H*-pyrimido[4,5*-b*]indole-4-carboxamide 7-fluoro-*N*,*N*,9-triméthyl-2-pyridin-4-yl-9*H*-pyrimido[4,5*-b*]indole-4-carboxamide 7-chloro-9-méthyl-2-pyridin-4-yl-4-(pyrrolidin-1-ylcarbonyl)-9*H*-pyrimido-[4,5*-b*]indole
7-chloro-*N*,*N*,9-triméthyl-2-pyridin-4-yl-9*H*-pyrimido[4,5*-b*]indole-4-carboxamide 7-chloro-*N*,*N*,9-triméthyl-2-pyridin-3-yl-9*H*-pyrimido[4,5*-b*]indole-4-carboxamide 7-chloro-*N*,*N*,9-triméthyl-2-pyridin-2-yi-9*H*-pyrimido[4,5*-b*]indole-4-carboxamide 7-chloro-*N*,*N*,9-triméthyl-2-pyrazin-2-yl-9*H*-pyrimido[4,5*-b*]indole-4-carboxamide 2-(7-chloro-9-méthyl-2-phényl-9H-pyrimido[4,5*-b*]indol-4-yl)-*N*,*N*-diméthyl-acétamide
2-(6-chloro-9-méthyl-2-phényl-9*H*-pyrimido[4,5*-b*]indol-4-yl)-*N*,*N*-diméthyl-acétamide
2-(7-chloro-9-méthyl-2-pyridin-4-yl-9*H*-pyrimido[4,5*-b*]indol-4-yl)-*N*,*N*-diméthyl-acétamide
2-(7-chloro-9-méthyl-2-pyridin-3-yl-9*H*-pyrimido[4,5*-b*]indol-4-yl)-*N*,*N*-diméthyl-acétamide
*N*,*N*-diméthyl-2-phényl-9*H*-pyrimido[4,5*-b*]indole-4-carboxamide 7-chloro-9-méthyl-2-phényl-9*H*-pyrimido[4,5*-b*]indole-4-carboxylate d'éthyle 7-chloro-9-méthyl-2-phényl-9*H*-pyrimido[4,5*-b*]indole-4-carboxylate de méthyle acide 7-chloro-9-méthyl-2-phényl-9*H*-pyrimido[4,5*-b*]indole-4-carboxylique 7-chloro-*N*,*N*,9-triméthyl-2-(6-méthylpyridin-3-yl)-9*H*-pyrimido[4,5*-b*]indole-4-carboxamide
7-chloro-2-(6-méthoxypyridin-3-yl)-*N*,*N*,9-triméthyl-9*H*-pyrimido[4,5*-b*]indole-4-carboxamide
7-chloro-*N*,*N*,9-triméthyl-2-(2-méthylpyridin-4-yl)-9*H*-pyrimido[4,5*-b*]indole-4-carboxamide
7-chloro-2-(2-méthoxypyridin-4-yl)-*N*,*N*,9-triméthyl-9*H*-pyrimido[4,5*-b*]indole-4-carboxamide
7-chloro-9-isobutyl-*N*,*N*-dim éthyl-2-pyridin-4-yl-9*H*-pyrimido[4,5*-b*]indole-4-carboxamide
7-chloro-2-cyclopropyl-*N*,*N*,9-triméthyl-9*H*-pyrimido[4,5*-b*]indole-4-carboxamide 7-chloro-*N*,*N*,2,9-tétraméthyl-9*H*-pyrimido[4,5*-b*]indole-4-carboxamide 7-chloro-2-isopropyl-*N*,*N*,9-triméthyl-9*H*-pyrimido[4,5*-b*]indole-4-carboxamide
7-chloro-*N*,*N*,9-triméthyl-2-(tétrahydro-2*H*-pyran-4-yl)-9*H*-pyrimido[4,5*-b*]indole-4-carboxamide
7-chloro-2-{4-[(diméthylamino)méthyl]phényl}-*N*,*N*,9-triméthyl-9*H*-pyrimido[4,5-b]indole-4-carboxamide
7-chloro-2-(6-chloropyridin-3-yl)-*N*,*N*,9-triméthyl-9*H*-pyrimido[4,5*-b*]indole-4-carboxamide
7-chloro-*N*,*N*,9-triméthyl-2-[6-(trifluorométhyl)pyridin-3-yl]-9*H*-pyrimido[4,5-b]indole-4-carboxamide
7-chloro-2-(6-éthoxypyridin-3-yl)-*N*,*N*,9-triméthyl-9*H*-pyrimido[4,5*-b*]indole-4-carboxamide
7-chloro-2-(6-éthylpyridin-3-yl)-*N*,*N*,9-triméthyl-9*H*-pyrimido[4,5*-b*]indole-4-carboxamide
7-chloro-2-(5-éthylpyridin-3-yl)-*N*,*N*,9-triméthyl-9*H*-pyrimido[4,5*-b*]indole-4-carboxamide
7-chloro-*N*,*N*,9-triméthyl-2-(5-méthylpyridin-3-yl)-9*H*-pyrimido[4,5*-b*]indole-4-carboxamide
7-chloro-*N*,*N*,9-triméthyl-2-pyrimidin-5-yl-9*H*-pyrimido[4,5*-b*]indole-4-carboxamide 7-chloro-2-(5-méthoxypyridin-3-yl)-*N*,*N*,9-triméthyl-9*H*-pyrimido[4,5*-b*]indole-4-carboxamide
7-chloro-2-[6-(méthoxyméthyl)pyridin-3-yl]-*N*,*N*,9-triméthyl-9*H*-pyrimido[4,5-*b*]indole-4-carboxamide
7-chloro-*N*,*N*,9-triméthyl-2-(2-méthyl-1,3-thiazol-4-yl)-9*H*-pyrimido[4,5*-b*]indole-4-carboxamide
7-chloro-2-{6-[(diméthylamino)méthyl]pyridin-3-yl}-*N*,*N*,9-triméthyl-9*H-*pyrimido[4,5*-b*]indole-4-carboxamide
7-chloro-2-(5,6-diméthylpyridin-3-yl)-*N*,*N*,9-triméthyl-9*H*-pyrimido[4,5*-b*]indole-4-carboxamide
7-chloro-*N*,*N*,9-triméthyl-2-(5,6,7,8-tétrahydroquinolin-3-yl)-9*H*-pyrimido[4,5-b]indole-4-carboxamide
7-chloro-2-(2,6-diméthylpyridin-4-yl)-*N*,*N*,9-triméthyl-9*H*-pyrimido[4,5*-b*]indole-4-carboxamide
à l'état de base ou de sel d'addition à un acide, ainsi qu'à l'état d'hydrate ou de solvate.

5. Procédé de préparation d'un composé de formule (I) selon l'une quelconque des revendications 1 à 4, comprenant l'étape consistant à cycliser le composé de formule générale (III), 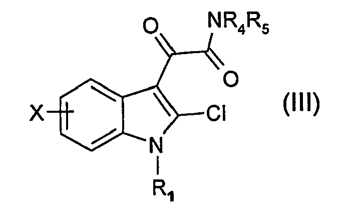 dans laquelle X R₁, R₄ et R₅ sont tels que définis dans la formule (I) selon la revendication 1,
en présence d'une amidine d'alkyle, d'hétéroalkyle, d'hétéroalkyle, d'aryle ou d'hétéroaryle de formule générale R₂C(NH)NH₂ dans laquelle R₂ est tel que défini dans la formule (I) selon la revendication 1, en chauffant dans un solvant apolaire ou polaire tel que par exemple du xylène ou de la *N*,*N*-diméthylformamide pour conduire à un composé de formule générale (I) dans laquelle n est égal à 0 et Y représente un groupe NR₄R₅.

6. Procédé de préparation d'un composé de formule (I) selon l'une quelconque des revendications 1 à 4, comprenant l'étape consistant à réaliser une réaction de carbonylation sur le composé de formule générale (VII), 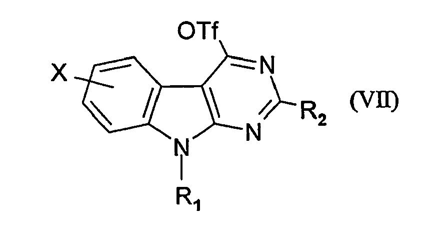 dans laquelle X, R₁ et R₂ sont tels que définis dans la formule (I) selon la revendication 1 et OTf représente un groupe triflate,
par exemple en présence d'un catalyseur tel que l'acétate de palladium, de monoxyde de carbone, de ligand de type phosphine tel que le 1,3-bis(diphényl-phosphinopropane) et d'un alcool de formule générale R₃OH, dans laquelle R₃ est tel que défini ci-dessus, pour obtenir le composé de formule générale (I) dans laquelle n est égal à 0 et Y représente un groupe OR₃.

7. Procédé de préparation d'un composé de formule (I) selon l'une quelconque des revendications 1 à 4, comprenant l'étape consistant à saponifier puis décarboxyler le composé de formule générale (IX), 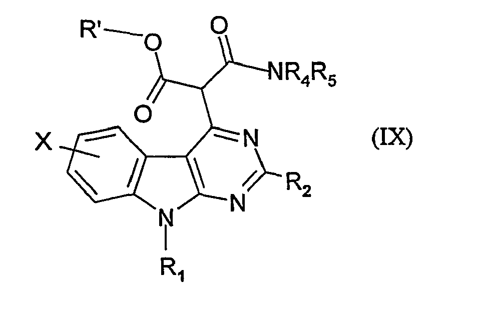 dans laquelle X, R₁, R₂, R₄ et R₅ sont tels que définis dans la formule (I) selon la revendication 1 et R' représente un (C₁-C₆)alkyle,
en utilisant par exemple de l'hydroxyde de lithium, dans un mélange de méthanol, d'eau et d'un solvant éthéré, pour obtenir le composé de formule générale (I) dans laquelle n est égal à 1 et Y représente un groupe NR₄R₅ .

8. Composé de formule (III) 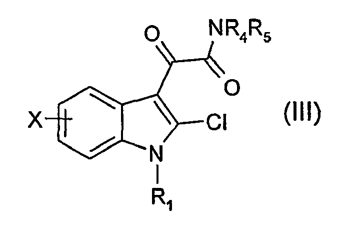 dans laquelle
X, R_{1,} R₄ et R₅ sont tels que définis dans la revendication 1.

9. Composé de formule (VII) 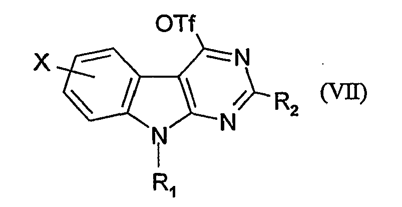 dans laquelle
OTf représente un groupe triflate ;
X, R₁ et R₂ étant tels que définis dans la revendication 1.

10. Composé de formule (IX) 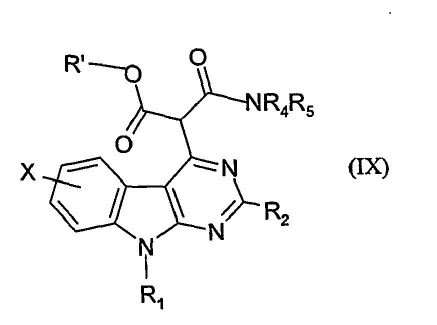 dans laquelle
X, R₁, R₂, R₄ et R₅ sont tels que définis dans la revendication 1 ; et
R' représente un (C₁-C₆)alkyle.

11. Médicament, **caractérisé en ce qu'**il comprend un composé de formule (I) selon l'une quelconque des revendications 1 à 4, ou un sel d'addition de ce composé à un acide pharmaceutiquement acceptable, ou encore un hydrate ou un solvate du composé de formule (I).

12. Composition pharmaceutique, **caractérisée en ce qu'**elle comprend un composé de formule (I) selon l'une quelconque des revendications 1 à 4, ou un sel pharmaceutiquement acceptable, un hydrate ou un solvate de ce composé, ainsi qu'au moins un excipient pharmaceutiquement acceptable.

13. Utilisation d'un composé de formule (I) selon l'une quelconque des revendications 1 à 4 pour la préparation d'un médicament destiné à prévenir ou à traiter les neuropathies périphériques, les affections du motoneurone, les maladies neurodégénératives du système nerveux central, l'anxiété, l'épilepsie, les troubles du sommeil, l'insuffisance rénale aiguë ou chronique, la glomérulonéphrite, la néphropathie diabétique, l'ischémie et l'insuffisance cardiaques, l'infarctus du myocarde, l'ischémie des membres inférieurs, le vasospasme coronaire, l'angine de poitrine, les pathologies associées aux valves cardiaques, les maladies cardiaques inflammatoires, les effets secondaires dus à des médicaments cardiotoxiques ou aux suites d'une chirurgie cardiaque, l'athérosclérose et ses complications thromboemboliques, la resténose, les rejets de greffes, les conditions liées à une prolifération ou une migration incorrectes des cellules musculaires lisses, les tumeurs et cancers, les stress cutanés, les maladies inflammatoires chroniques, notamment la polyarthrite rhumatoïde, et les maladies inflammatoires pulmonaires.

## Claims

1. Compound corresponding to the formula (I): 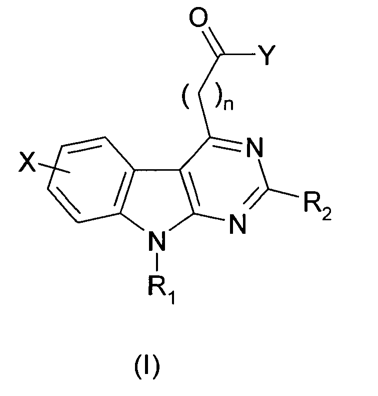 in which:
n represents the number 0 or 1;
X represents a hydrogen or halogen atom;
Y represents an OR₃ group or an NR₄R₅ group;
R₁ represents a hydrogen atom or a (C₁-C₆) alkyl group;
R₂ represents a (C₁-C₆) alkyl group, a phenyl or a monocyclic or bicyclic heterocycle, said phenyl or heterocycle groups possibly bearing one or more halogen atoms and/or one or more (C₁-C₆) alkyl, (C₁-C₆) alkoxyl, (C₁-C₆) alkoxy (C₁-C₆) alkyl, (C₁-C₆) alkylamino (C₁-C₆)alkyl or (C₁-C₆)dialkylamino(C₁-C₆)alkyl groups;
R₃ represents a hydrogen atom, a (C₁-C₆)alkyl group or a benzyl; and
R₄ and R₅ each represent, independently of one another, a hydrogen atom or a (C₁-C₆)alkyl group,
or else R₄ and R₅ form, with the nitrogen atom that they bear, an aziridinyl, azetidinyl, pyrrolidinyl, piperidinyl, morpholinyl,
thiomorpholinyl or piperazinyl, optionally substituted by a (C₁-C₆) alkyl, phenyl or
heterocycle;
in base or acid addition salt form, and also in the hydrate or solvate form.

2. Compound of formula (I) according to Claim 1, **characterized in that**:
n represents the number 0 or 1;
X represents a hydrogen or halogen atom;
Y represents an OR₃ group or an NR₄R₅ group;
R₁ represents a hydrogen atom or a (C₁-C₆)alkyl group;
R₂ represents a phenyl or a heterocycle of pyridinyl, pyrimidinyl, pyrazinyl or pyridazinyl type, the phenyl or heterocycle possibly bearing one or more halogen atoms and/or one or more (C₁-C₆)alkyl or (C₁-C₆)alkoxyl groups;
R₃ represents a hydrogen atom, a (C₁-C₆)alkyl or a benzyl; and
R₄ and R₅ each represent, independently of one another, a hydrogen atom or a (C₁-C₆)alkyl group, or else R₄ and R₅ form, with the nitrogen atom that they bear, an aziridinyl, azetidinyl, pyrrolidinyl, piperidinyl, morpholinyl, thiomorpholinyl or piperazinyl group, optionally substituted by a (C₁-C₆)alkyl, phenyl or azetidinyl;
in base or acid addition salt form, and also in the hydrate or solvate form.

3. Compound of formula (I) according to Claim 1, **characterized in that**:
n represents the number 0 or 1;
X represents a hydrogen or fluorine or chlorine atom;
Y represents a hydroxy, OCH₃ , O(CH₂CH₃), N(CH₃)₂, N(CH₂CH₃)₂, pyrrolidinyl, piperidinyl, morpholinyl, (*N*-azetidinyl)piperidinyl or *N*-methylpiperazinyl group;
R₁ represents a hydrogen atom, a methyl or isobutyl group; and
R₂ represents a phenyl, a methyl, isopropyl or cyclopropyl group or a heterocycle of pyridinyl, pyrazinyl, pyrimidinyl, thiazolyl, tetrahydroquinolinyl or tetrahydropyranyl type, the phenyl and heterocycle groups possibly optionally being substituted by one or more halogen groups and/or one or more (C₁-C₆)alkyl, (C₁-C₆) alkoxyl, (C₁-C₆) alkoxy (C₁-C₆) alkyl, (C₁-C₆)dialkylamino or (C₁-C₆)dialkylamino(C₁-C₆)alkyl groups;
in the base or acid addition salt form, and also in the hydrate or solvate form.

4. Compound of formula (I) according to Claim 1 or 3, chosen from:
*N*,*N*,9-trimethyl-2-phenyl-9*H*-pyrimido[4,5*-b*]indole-4-carboxamide;
7-*chloro*-*N*,*N*,9-trimethyl-2-phenyl-9*H*-pyrimido[4,5-*b*]indole-4-carboxamide;
7-*fluoro*-*N*,*N*-9-trimethyl-2-phenyl-9*H*-pyrimido[4,5-b]indole-4-carboxamide;
6-*chloro*-*N*,*N*-9-trimethyl-2-phenyl-9*H*-pyrimido[4,5-b]indole-4-carboxamide;
7-chloro-*N*,*N*-dimethyl-2-phenyl-9*H*-pyrimido[4,5-b]indole-4-carboxamide;
7-chloro-*N*,*N*-diethyl-9-methyl-2-phenyl-9*H-*pyrimido[4,5*-b*]indole-4-carboxamide;
7-chloro-9-methyl-2-phenyl-4-(pyrrolidin-1-ylcarbonyl)-9*H*-pyrimido[4,5*-b*]indole;
7-chloro-9-methyl-2-phenyl-4-(piperidin-1-ylcarbonyl)-9*H*-pyrimido[4,5*-b*]indole;
7-chloro-9-methyl-4-(morpholin-4-ylcarbonyl)-2-phenyl-9*H*-pyrimido[4,5*-b*]indole;
4-[(4-azetidin-1-yl)piperidin-1-ylcarbonyl]-7-chloro-9-methyl-2-phenyl-9*H*-pyrimido-[4,5-*b*]indole;
7-chloro-9-methyl-4-[(4-methylpiperazin-1-yl)carbonyl]-2-phenyl-9*H*-pyrimido[4,5*-b*]indole;
6-chloro-*N*,*N*-9-trimethyl-2-pyridin-4-yl-9*H-*pyrimido[4,5*-b*]indole-4-carboxamide;
7-fluoro-*N*,*N*,9-trimethyl-2-pyridin-4-yl-9*H-*pyrimido[4,5*-b*]indole-4-carboxamide;
7-chloro-9-methyl-2-pyridin-4-yl-4-(pyrrolidin-1-ylcarbonyl)-9*H*-pyrimido-[4,5*-b*]indole;
7-chloro-*N*,*N*,9-trimethyl-2-pyridin-4-yl-9*H-*pyrimido[4,5*-b*]indole-4-carboxamide;
7-chloro-*N*,*N*,9-trimethyl-2-pyridin-3-yl-9*H-*pyrimido[4,5*-b*]indole-4-carboxamide;
7-chloro-*N*,*N*-9-trimethyl-2-pyridin-2-yl-9*H-*pyrimido[4,5*-b*]indole-4-carboxamide 7-chloro-*N*,*N*-9-trimethyl-2-pyrazin-2-yl-9*H-*pyrimido[4,5*-b*]indole-4-carboxamide;
2-(7-chloro-9-methyl-2-phenyl-9*H*-pyrimido[4,5-*b*]indol-4-yl)-*N*,*N*-dimethylacetamide;
2-(6-chloro-9-methyl-2-phenyl-9*H*-pyrimido]4,5-*b*]indol-4-yl)-*N*,*N*-dimethylacetamide;
2-(7-chloro-9-methyl-2-pyridin-4-yl-9*H-*pyrimido[4,5*-b*]indol-4-yl)-*N*,*N*-dimethylacetamide;
2-(7-chloro-9-methyl-2-pyridin-3-yl-9*H-*pyrimido[4,5*-b*]indol-4-yl)-*N*,*N*-dimethylacetamide ;
*N*,*N*-dimethyl-2-phenyl-9*H*-pyrimido[4,5*-b*]indole-4-carboxamide;
ethyl 7-chloro-9-methyl-2-phenyl-9*H*-pyrimido[4,5-b]indole-4-carboxylate;
methyl 7-chloro-9-methyl-2-phenyl-9*H*-pyrimido[4,5-b]indole-4-carboxylate;
7-chloro-9-methyl-2-phenyl-9*H*-pyrimido[4,5-*b*]indole-4-carboxylic acid;
7-chloro-*N*,*N*-9-trimethyl-2-(6-methylpyridin-3-yl)-9*H*-pyrimido[4,5*-b*]indole-4-carboxamide;
7-chloro-2-(6-methoxypyridin-3-yl)-*N*,*N*,9-trimethyl-9*H*-pyrimido[4,5*-b*]indole-4-carboxamide;
7-chloro-*N*,*N*-9-trimethyl-2-(2-methylpyridin-4-yl)-9*H*-pyrimido[4,5*-b*]indole-4-carboxamide;
7-chloro-2-(2-methoxypyridin-4-yl)-*N*,*N*,9-trimethyl-9*H*-pyrimido[4,5*-b*]indole-4-carboxamide;
7-chloro-9-isobutyl-*N*,*N*-dimethyl-2-pyridin-4-yl-9*H*-pyrimido[4,5*-b*]indole-4-carboxamide;
7-chloro-2-cyclopropyl-*N*,*N*,9-trimethyl-9*H-*pyrimido[4,5*-b*]indole-4-carboxamide;
7-chloro-*N*,*N*,2,9-tetramethyl-9*H*-pyrimido[4,5-*b*]indole-4-carboxamide;
7-chloro-2-isopropyl-*N*,*N*,9-trimethyl-9*H-*pyrimido[4,5*-b*]indole-4-carboxamide;
7-chloro-*N*,*N*,9-trimethyl-2-(tetrahydro-2*H*-pyran-4-yl)-9*H*-pyrimido[4,5*-b*]indole-4-carboxamide;
7-chloro-2-{4-[(dimethylamino)methyl]phenyl}-*N*,*N*,9-trimethyl-9*H*-pyrimido[4,5*-b*]indole-4-carboxamide;
7-chloro-2-(6-chloropyridin-3-yl)-*N*,*N*,9-trimethyl-9*H*-pyrimido[4,5*-b*]indole-4-carboxamide;
7-chloro-*N*,*N*,9-trimethyl-2-[6-(trifluoromethyl)pyridin-3-yl]-9*H*-pyrimido[4,5-*b*]indole-4-carboxamide;
7-chloro-2-(6-ethoxypyridin-3-yl)-*N*,*N*,9-trimethyl-9*H*-pyrimido[4,5*-b*]indole-4-carboxamide;
7-chloro-2-(6-ethylpyridin-3-yl)-*N*,*N*,9-trimethyl-9*H*-pyrimido[4,5*-b*]indole-4-carboxamide;
7-chloro-2-(5-ethylpyridin-3-yl)-*N*,*N*,9-trimethyl-9*H*-pyrimido[4,5*-b*]indole-4-carboxamide;
7-chloro-*N*,*N*,9-trimethyl-2-(5-methylpyridin-3-yl)-9*H*-pyrimido[4,5*-b*]indole-4-carboxamide;
7-chloro-*N*,*N*,9-trimethyl-2-pyrimidin-5-yl-9*H-*pyrimido[4,5*-b*]indole-4-carboxamide;
7-chloro-2-(5-methoxypyridin-3-yl)-*N*,*N*,9-trimethyl-9*H*-pyrimido[4,5*-b*]indole-4-carboxamide;
7-chloro-2-[6-(methoxymethyl)pyridin-3-yl]-*N*,*N*,9-trimethyl-9*H*-pyrimido[4,5*-b*]indole-4-carboxamide;
7-chloro-*N*,*N*,9-trimethyl-2-(2-methyl-1,3-thiazol-4-yl)-9*H*-pyrimido[4,5*-b*]indole-4-carboxamide;
7-chloro-2-{6-[(dimethylamino)methyl]pyridin-3-yl}-*N*,*N*,9-trimethyl-9*H*-pyrimido[4,5*-b*]indole-4-carboxamide;
7-chloro-2-(5,6-dimethylpyridin-3-yl)-*N*,*N*,9-trimethyl-9*H*-pyrimido[4,5*-b*]indole-4-carboxamide;
7-chloro-*N*,*N*,9-trimethyl-2-(5,6,7,8-tetrahydroquinolin-3-yl)9*H*-pyrimido[4,5*-b*]indole-4-carboxamide;
7-chloro-2-(2,6-dimethylpyridin-4-yl)-*N*,*N*,9-trimethyl-9*H*-pyrimido[4,5*-b*]indole-4-carboxamide;
in base or acid addition salt form and also in the hydrate or solvate form.

5. Process for preparing a compound of formula (I) according to any one of Claims 1 to 4, comprising the step that consists in:
cyclizing the compound of general formula (III): 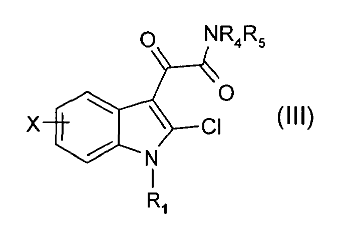 in which X, R₁, R₄ and R₅ are as defined in the formula (I) according to Claim 1,
in the presence of an alkyl, heteroalkyl, heteroalkyl, aryl or heteroaryl amidine of general formula R₂C(NH)NH₂ in which R₂ is as defined in the formula (I) according to Claim 1, by heating in an apolar or polar solvent such as, for example, xylene or *N*,*N*-dimethylformamide in order to result in a compound of general formula (I) in which n is equal to 0 and Y represents an NR₄R₅ group.

6. Process for preparing a compound of formula (I) according to any one of Claims 1 to 4, comprising the step consisting in carrying out a carbonylation reaction on the compound of general formula (VII): 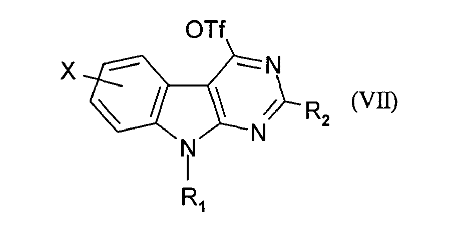 in which X, R₁ and R₂ are as defined in the formula (I) according to Claim 1 and OTf represents a triflate group,
for example in the presence of a catalyst such as palladium acetate, of carbon monoxide, of a ligand of phosphine type such as 1,3-bis(diphenylphosphino)propane and an alcohol of general formula R₃OH, in which R₃ is as defined above, in order to obtain the compound of general formula (I) in which n is equal to 0 and Y represents an OR₃ group.

7. Process for preparing a compound of formula (I) according to any one of Claims 1 to 4, comprising the step consisting in:
saponifying then decarboxylating the compound of general formula (IX): 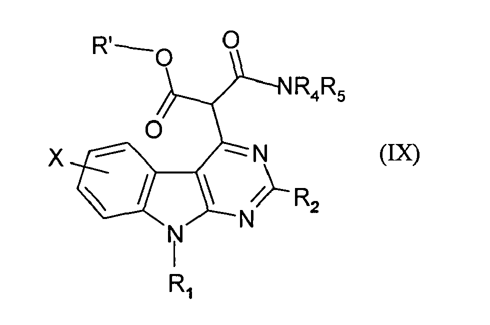 in which X, R₁, R₂, R₄ and R₅ are as defined in the formula (I) according to Claim 1 and R' represents a (C₁-C₆) alkyl,
by using for example lithium hydroxide, in a mixture of methanol, water and an ethereal solvent, in order to obtain the compound of general formula (I) in which n is equal to 1 and Y represents an NR₄R₅ group.

8. Compound of formula (III): 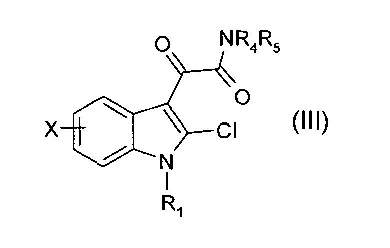 in which
X, R₁, R₄ and R₅ are as defined in Claim 1.

9. Compound of formula (VII): 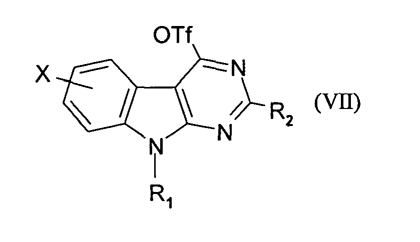 in which
OTf represents a triflate group; and X, R₁ and R₂ being as defined in Claim 1.

10. Compound of formula (IX): 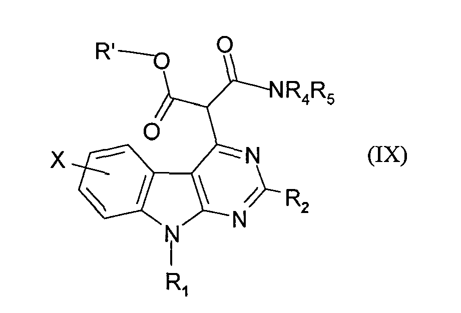 in which
X, R₁, R₂, R₄ and R₅ are as defined in Claim 1; and
R' represents a (C₁-C₆)alkyl.

11. Medication, **characterized in that** it comprises a compound of formula (I) according to any one of Claims 1 to 4, or a pharmaceutically acceptable acid addition salt of this compound, or else a hydrate or solvate of the compound of formula (I).

12. Pharmaceutical composition, **characterized in that** it comprises a compound of formula (I) according to any one of Claims 1 to 4, or a pharmaceutically acceptable salt, a hydrate or a solvate of this compound, and also at least one pharmaceutically acceptable excipient.

13. Use of a compound of formula (I) according to any one of Claims 1 to 4 to prepare a medication intended to prevent or treat periphery neuropathies, motor neurone diseases, neurodegenerative diseases of the central nervous system, anxiety, epilepsy, sleep disorders, acute or chronic renal failure, glomerulonephritis, diabetic nephropathy, cardiac ischemia and heart failure, myocardial infarction, ischemia of the lower limbs, coronary vasospasm, angina poitrineous, pathologies associated with the heart valves, inflammatory heart diseases, secondary effects due to cardiotoxic medication or as a result of heart surgery, atherosclerosis and its thromboembolic complications, restenosis, graft rejections, conditions related to an incorrect proliferation or migration of smooth muscle cells, tumors and cancers, cutaneous stress, chronic inflammatory diseases, especially rheumatoid arthritis, and pulmonary inflammatory diseases.

## Patentansprüche

1. Verbindung der Formel (I) 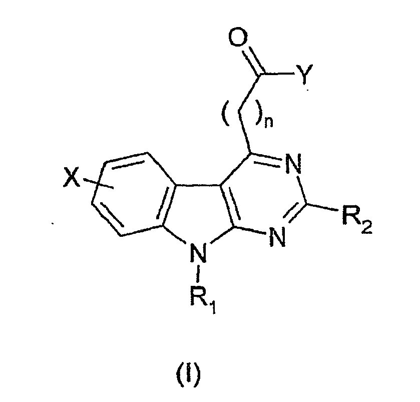 worin
n für die Zahl 0 oder 1 steht;
X für ein Wasserstoff- oder Halogenatom steht;
Y für eine OR₃-Gruppe oder eine NR₄R₅-Gruppe steht;
R₁ für ein Wasserstoffatom oder eine (C₁-C₆)-Alkylgruppe steht;
R₂ für eine (C₁-C₆)-Alkylgruppe, Phenyl oder einen monocyclischen oder bicyclischen Heterocyclus steht, wobei die Phenyl- oder Heterocyclusgruppen ein oder mehrere Halogenatome und/oder eine oder mehrere (C₁-C₆)-Alkyl-, (C₁-C₆)-Alkoxy-, (C₁-C₆)-Alkoxy- (C₁-C₆) -alkyl-, (C₁-C₆) -Alkylamino- (C₁-C₆) - alkyl- oder (C₁-C₆)-Dialkylamino- (C₁-C₆)-alkylgruppen tragen können;
R₃ für ein Wasserstoffatom, eine (C₁-C₆)-Alkylgruppe oder Benzyl steht;
R₄ und R₅ jeweils unabhängig voneinander für ein Wasserstoffatom oder eine (C₁-C₆)-Alkylgruppe stehen oder R₄ und R₅ mit dem Stickstoffatom, an das sie gebunden sind, eine Aziridinyl-, Azetidinyl-, Pyrrolidinyl-, Piperidinyl-, Morpholinyl-, Thiomorpholinyl- oder Piperazinylgruppe, die gegebenenfalls durch (C₁-C₆)-Alkyl, Phenyl oder Heterocyclus substituiert ist, bilden; in Basen- oder Säureadditionssalzform sowie in Hydrat- oder Solvatform.

2. Verbindung der Formel (I) nach Anspruch 1, **dadurch gekennzeichnet, daß**:
n für die Zahl 0 oder 1 steht;
X für ein Wasserstoff- oder Halogenatom steht;
Y für eine OR₃-Gruppe oder eine NR₄R₅-Gruppe steht;
R₁ für ein Wasserstoffatom oder eine (C₁-C₆)-Alkylgruppe steht;
R₂ für Phenyl oder einen Heterocyclus vom Pyridinyl-, Pyrimidinyl-, Pyrazinyl- oder Pyridazinyltyp steht, wobei das Phenyl oder der Heterocyclus ein oder mehrere Halogenatome und/oder eine oder mehrere (C₁-C₆)-Alkyl- und/oder (C₁-C₆)-Alkoxygruppen tragen können; R₃ für ein Wasserstoffatom, eine (C₁-C₆)-Alkylgruppe oder Benzyl steht; und
R₄ und R₅ jeweils unabhängig voneinander für ein Wasserstoffatom oder eine (C₁-C₆)-Alkylgruppe stehen oder R₄ und R₅ mit dem Stickstoffatom, an das sie gebunden sind, eine Aziridinyl-, Azetidinyl-, Pyrrolidinyl-, Piperidinyl-, Morpholinyl-, Thiomorpholinyl- oder Piperazinylgruppe, die gegebenenfalls durch (C₁-C₆)-Alkyl, Phenyl oder Azetidinyl substituiert ist, bilden; in Basen- oder Säureadditionssalzform sowie in Hydrat- oder Solvatform.

3. Verbindung der Formel (I) nach Anspruch 1, **dadurch gekennzeichnet, daß**:
n für die Zahl 0 oder 1 steht;
X für ein Wasserstoff- oder Fluor- oder Chloratom steht;
Y für eine Hydroxy-, OCH₃-, O (CH₂CH₃) -, N (CH₃)₂- , N(CH₂CH₃)₂-, Pyrrolidinyl-, Piperidinyl-, Morpholinyl-, (*N*-Azetidinyl)piperidinyl- oder *N-*Methylpiperazinylgruppe steht;
R₁ für ein Wasserstoffatom oder eine Methyl- oder Isobutylgruppe steht;
R₂ für Phenyl, eine Methyl-, Isopropyl- oder Cyclopropylgruppe oder einen Heterocyclus vom Pyridinyl-, Pyrazinyl-, Pyrimidinyl-, Thiazolyl-, Tetrahydrochinolinyl- oder Tetrahydropyranyltyp steht, wobei die Phenyl- und Heterocyclusgruppen gegebenenfalls durch eine oder mehrere Halogengruppen und/oder eine oder mehrere (C₁-C₆)-Alkyl-, (C₁-C₆) -Alkoxy-, (C₁-C₆) -Alkoxy- (C₁-C₆) - alkyl-, (C₁-C₆)-Dialkylamino- oder (C₁-C₆)-Dialkylamino-(C₁-C₆)-alkylgruppen substituiert sein können;
in Basen- oder Säureadditionssalzform sowie in Hydrat- oder Solvatform.

4. Verbindung der Formel (I) nach Anspruch 1 oder 3, ausgewählt unter:
*N*,*N*,9-Trimethyl-2-phenyl-9*H*-pyrimido[4,5*-b*]indol-4-carboxamid;
7-Chlor-*N*,*N*,9-trimethyl-2-phenyl-9*H*-pyrimido[4,5-*b*] indol-4-carboxamid;
7-Fluor-*N*,*N*-9-trimethyl-2-phenyl-9*H*-pyrimido[4,5-*b*] indol-4-carboxamid;
6-Chlor-*N*,*N*-9-trimethyl-2-phenyl-9*H*-pyrimido[4,5-*b*] indol-4-carboxamid;
7-Chlor-*N*,*N*-dimethyl-2-phenyl-9*H*-pyrimido[4,5-*b*] indol-4-carboxamid;
7-Chlor-*N*,*N*-diethyl-9-methyl-2-phenyl-9*H*-pyrimido-[4,5*-b*]indol-4-carboxamid;
7-Chlor-9-methyl-2-phenyl-4-(pyrrolidin-1-yl-carbonyl)-9*H*-pyrimido[4,5*-b*]indol;
7-Chlor-9-methyl-2-phenyl-4-(piperidin-1-yl-carbonyl)-9*H*-pyrimido[4,5*-b*]indol;
7-Chlor-9-methyl-4-(morpholin-4-ylcarbonyl)-2-phenyl-9*H*-pyrimido[4,5*-b*]indol;
4-[(4-Azetidin-1-yl)piperidin-1-ylcarbonyl]-7-chlor-9-methyl-2-phenyl-9*H*-pyrimido-[4,5*-b*]indol;
7-Chlor-9-methyl-4-[(4-methylpiperazin-1-yl)-carbonyl]-2-phenyl-9*H*-pyrimido[4,5*-b*]indol;
6-Chlor-*N*,*N*-9-trimethyl-2-pyridin-4-yl-9*H-*pyrimido[4,5*-b*]indol-4-carboxamid;
7-Fluor-*N*,*N*,9-trimethyl-2-pyridin-4-yl-9*H-*pyrimido[4,5*-b*]indol-4-carboxamid;
7-Chlor-9-methyl-2-pyridin-4-yl-4-(pyrrolidin-1-ylcarbonyl)-9*H*-pyrimido-[4,5*-b*]indol;
7-Chlor-*N*,*N*,9-trimethyl-2-pyridin-4-yl-9*H-*pyrimido[4,5*-b*]indol-4-carboxamid;
7-Chlor-*N*,*N*,9-trimethyl-2-pyridin-3-yl-9*H-*pyrimido[4,5*-b*]indol-4-carboxamid;
7-Chlor-*N*,*N*-9-trimethyl-2-pyridin-2-yl-9*H-*pyrimido[4,5*-b*]indol-4-carboxamid;
7-Chlor-*N*,*N*-9-trimethyl-2-pyrazin-2-yl-9*H-*pyrimido[4,5*-b*]indol-4-carboxamid;
2-(7-Chlor-9-methyl-2-phenyl-9*H*-pyrimido[4,5*-b*]-indol-4-yl)-*N*,*N*-dimethylacetamid;
2-(6-Chlor-9-methyl-2-phenyl-9*H*-pyrimido]4,5*-b*]-indol-4-yl)-*N*,*N*-dimethylacetamid;
2-(7-Chlor-9-methyl-2-pyridin-4-yl-9*H*-pyrimido-[4,5*-b*]indol-4-yl)-*N*,*N*-dimethylacetamid;
2-(7-Chlor-9-methyl-2-pyridin-3-yl-9*H*-pyrimido-[4,5*-b*]indol-4-yl)-*N*,*N*-dimethylacetamid;
*N*,*N*-Dimethyl-2-phenyl-9*H*-pyrimido[4,5*-b*]indol-4-carboxamid;
7-Chlor-9-methyl-2-phenyl-9*H*-pyrimido[4,5*-b*]indol-4-carbonsäureethylester;
7-Chlor-9-methyl-2-phenyl-9*H*-pyrimido[4,5*-b*]indol-4-carbonsäuremethylester;
7-Chlor-9-methyl-2-phenyl-9*H*-pyrimido[4,5*-b*]indol-4-carbonsäure;
7-Chlor-*N*,*N*-9-trimethyl-2-(6-methylpyridin-3-yl)-9*H*-pyrimido[4,5*-b*]indol-4-carboxamid;
7-Chlor-2-(6-methoxypyridin-3-yl)-*N*,*N*,9-trimethyl-9*H*-pyrimido[4,5*-b*]indol-4-carboxamid;
7-Chlor-*N*,*N*-9-trimethyl-2-(2-methylpyridin-4-yl)-9*H*-pyrimido[4,5*-b*]indol-4-carboxamid;
7-Chlor-2-(2-methoxypyridin-4-yl)-*N*,*N*,9-trimethyl-9*H*-pyrimido[4,5*-b*]indol-4-carboxamid;
7-Chlor-9-isobutyl-*N*,*N*-dimethyl-2-pyridin-4-yl-9*H-*pyrimido[4,5*-b*]indol-4-carboxamid;
7-Chlor-2-cyclopropyl-*N*,*N*,9-trimethyl-9*H-*pyrimido[4,5*-b*]indol-4-carboxamid;
7-Chlor-*N*,*N*,2,9-tetramethyl-9*H*-pyrimido[4,5*-b*]-indol-4-carboxamid;
7-Chlor-2-isopropyl-*N*,*N*,9-trimethyl-9*H*-pyrimido-[4,5*-b*]indol-4-carboxamid;
7-Chlor-*N*,*N*,9-trimethyl-2-(tetrahydro-2*H*-pyran-4-yl)-9*H*-pyrimido[4,5*-b*]indol-4-carboxamid;
7-Chlor-2-{4-[(dimethylamino)methyl]phenyl}-*N*,*N*,9-trimethyl-9*H*-pyrimido[4,5*-b*]indol-4-carboxamid;
7-Chlor-2-(6-chlorpyridin-3-yl)-*N*,*N*,9-trimethyl-9*H*-pyrimido[4,5*-b*]indol-4-carboxamid;
7-Chlor-*N*,*N*,9-trimethyl-2-[6-(trifluormethyl)-pyridin-3-yl]-9*H*-pyrimido[4,5*-b*]indol-4-carboxamid;
7-Chlor-2-(6-ethoxypyridin-3-yl)-*N*,*N*,9-trimethyl-9*H*-pyrimido[4,5*-b*]indol-4-carboxamid;
7-Chlor-2-(6-ethylpyridin-3-yl)-*N*,*N*,9-trimethyl-9*H*-pyrimido[4,5*-b*]indol-4-carboxamid;
7-Chlor-2-(5-ethylpyridin-3-yl)-*N*,*N*,9-trimethyl-9*H*-pyrimido[4,5*-b*]indol-4-carboxamid;
7-Chlor-*N*,*N*,9-trimethyl-2-(5-methylpyridin-3-yl)-9*H*-pyrimido[4,5*-b*]indol-4-carboxamid;
7-Chlor-*N*,*N*,9-trimethyl-2-pyrimidin-5-yl-9*H-*pyrimido[4,5*-b*]indol-4-carboxamid;
7-Chlor-2-(5-methoxypyridin-3-yl)-*N*,*N*,9-trimethyl-9*H*-pyrimido[4,5*-b*]indol-4-carboxamid;
7-Chlor-2-[6-(methoxymethyl)pyridin-3-yl]-*N*,*N*,9-trimethyl-9*H*-pyrimido[4,5*-b*]indol-4-carboxamid;
7-Chlor-*N*,*N*,9-trimethyl-2-(2-methyl-1,3-thiazol-4-yl)-9*H*-pyrimido[4,5*-b*]indol-4-carboxamid;
7-Chlor-2-{6-[(dimethylamino)methyl]pyridin-3-yl}-*N*,*N*,9-trimethyl-9*H*-pyrimido[4,5*-b*]indol-4-carboxamid;
7-Chlor-2-(5,6-dimethylpyridin-3-yl)-*N*,*N*,9-trimethyl-9*H*-pyrimido[4,5*-b*]indol-4-carboxamid;
7-Chlor-*N*,*N*,9-trimethyl-2-(5,6,7,8-tetrahydro-chinolin-3-yl)9*H*-pyrimido[4,5*-b*]indol-4-carboxamid;
7-Chlor-2-(2,6-dimethylpyridin-4-yl)-*N*,*N*,9-trimethyl-9*H*-pyrimido[4,5*-b*]indol-4-carboxamid;
in Basen- oder Säureadditionssalzform sowie in Hydrat- oder Solvatform.

5. Verfahren zur Herstellung einer Verbindung der Formel (I) nach einem der Ansprüche 1 bis 4, bei dem man
die Verbindung der allgemeinen Formel III 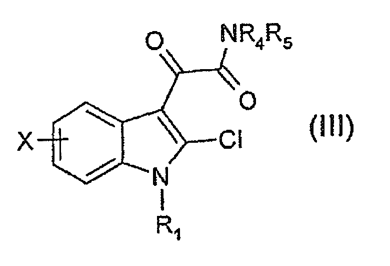 worin X, R₁, R₄ und R₅ die in Formel (I) gemäß Anspruch 1 angegebene Bedeutung besitzen,
in Gegenwart eines Alkyl-, Heteroalkyl-, Heteroalkyl-, Aryl- oder Heteroarylamidins der allgemeinen Formel R₂C(NH)NH₂, worin R₂ die in Formel (I) gemäß Anspruch 1 angegebene Bedeutung besitzt, durch Erhitzen in einem apolaren oder polaren Lösungsmittel wie beispielsweise Xylol oder *N*,*N*-Dimethylformamid zu einer Verbindung der allgemeinen Formel (I), worin n gleich 0 ist und Y für eine NR₄R₅-Gruppe steht, cyclisiert.

6. Verfahren zur Herstellung einer Verbindung der Formel (I) nach einem der Ansprüche 1 bis 4, bei dem man an der Verbindung der allgemeinen Formel (VII) 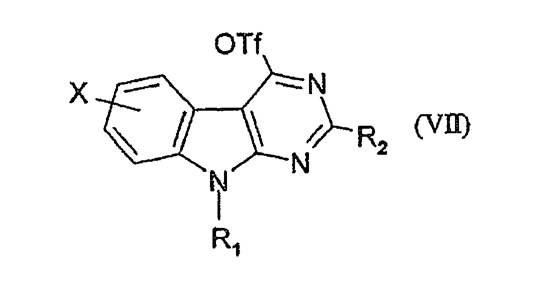 worin X, R₁ und R₂ die in Formel (I) gemäß Anspruch 1 angegebene Bedeutung besitzen und OTf für eine Triflatgruppe steht, eine Carbonylierungsreaktion durchführt,
beispielsweise in Gegenwart von einem Katalysator wie Palladiumacetat, Kohlenmonoxid, einem Liganden vom Phosphintyp wie 1,3-Bis(diphenylphosphinopropan) und einem Alkohol der allgemeinen Formel R₃OH, worin R₃ die oben angegebene Bedeutung besitzt, wobei man die Verbindung der allgemeinen Formel (I), in der n gleich 0 ist und Y für eine OR₃-Gruppe steht, erhält.

7. Verfahren zur Herstellung einer Verbindung der Formel (I) nach einem der Ansprüche 1 bis 4, bei dem man
die Verbindung der allgemeinen Formel (IX) 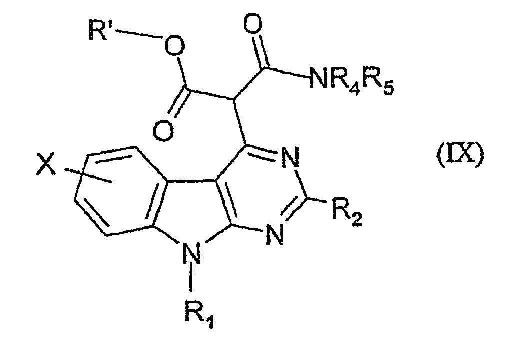 worin X, R₁, R₂, R₄ und R₅ die in Formel (I) gemäß Anspruch 1 angegebene Bedeutung besitzen und R' für (C₁-C₆)-Alkyl steht, verseift und dann decarboxyliert,
beispielsweise unter Verwendung von Lithiumhydroxid in einer Mischung von Methanol, Wasser und einem Ether-Lösungsmittel, wobei man die Verbindung der allgemeinen Formel (I), in der n gleich 1 ist und Y für eine NR₄R₅-Gruppe steht, erhält.

8. Verbindung der Formel (III) 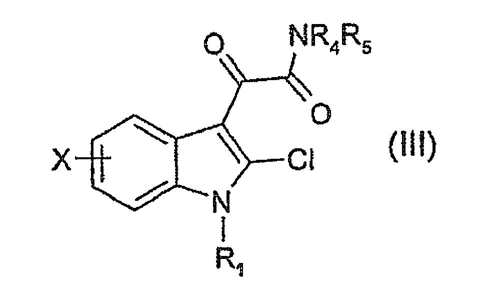 worin
X, R₁, R₄ und R₅ die in Anspruch 1 angegebene Bedeutung besitzen.

9. Verbindung der Formel (VII) 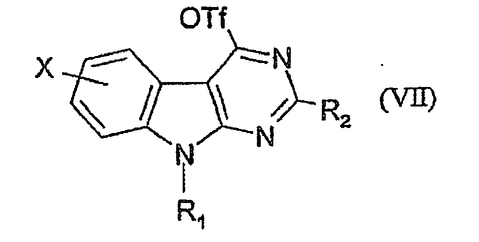 worin
OTf für eine Triflatgruppe steht und
X, R₁ und R₂ die in Anspruch 1 angegebene Bedeutung besitzen.

10. Verbindung der Formel (IX) 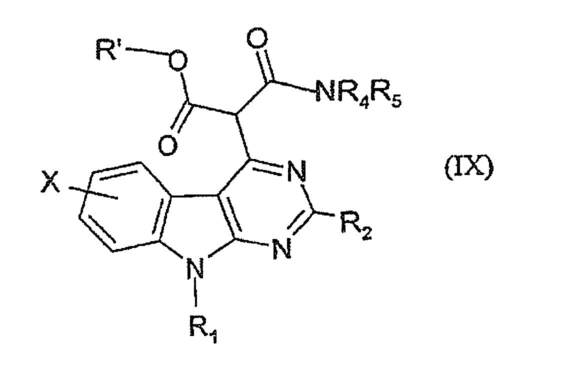 worin
X, R₁, R₂, R₄ und R₅ die in Anspruch 1 angegebene Bedeutung besitzen und
R' für (C₁-C₆)-Alkyl steht.

11. Arzneimittel, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure oder auch ein Hydrat oder ein Solvat der Verbindung der Formel (I) umfaßt.

12. Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz, ein Hydrat oder ein Solvat dieser Verbindung sowie mindestens einen pharmazeutisch unbedenklichen Trägerstoff umfaßt.

13. Verwendung einer Verbindung der Formel (I) nach einem der Ansprüche 1 bis 4 zur Herstellung eines Arzneimittels zur Prävention oder Behandlung von peripheren Neuropathien, Motoneuronenleiden, neurodegenerativen Erkrankungen des Zentralnervensystems, Angst, Epilepsie, Schlafstörungen, akuter oder chronischer Niereninsuffizienz, Glomerulonephritis, diabetischer Nephropathie, Herzischämie, Herzinsuffizienz, Myokardinfarkt, Ischämie der unteren Extremitäten, koronarem Gefäßspasmus, Angina pectoris, mit Herzklappen assoziierten Pathologien, entzündlichen Herzerkrankungen, Nebenwirkungen von kardiotoxischen Arzneimitteln oder nach Herzchirurgie, Atherosklerose und deren thromboembolischen Komplikationen, Restenose, Transplantatabstoßungen, mit inkorrekter Proliferation oder Migration von glatten Muskelzellen verbundenen Zuständen, Tumoren und Krebs, Hautstreß, chronischen entzündlichen Erkrankungen, insbesondere rheumatoider Arthritis, und entzündlichen Lungenerkrankungen.
